# EUROPÄISCHE PATENTANMELDUNG

(11) **EP 4 806 334 A2**
(43) Veröffentlichungstag der Anmeldung: **16.09.2026**
(21) Anmeldenummer: 26157673.0
(22) Anmeldetag: 10.02.2026
(51) Int. Cl.: A61B 1/00, A61B 1/05, A61B 1/307, A61B 1/015

(54) **RESEKTOSKOPVORRICHTUNG, RESEKTIONSWERKZEUG UND RESEKTOSKOP**

(30) Priorität: 24.02.2025 DE 102025106809
(71) Anmelder: Karl Storz SE & Co. KG, 78532 Tuttlingen (DE)
(72) Erfinder: SCHWARZ, Peter, 78532 Tuttlingen (DE); GRAF, Christian, 78532 Tuttlingen (DE)

(57) **Zusammenfassung**

Die vorliegende Erfindung betrifft eine Resektoskopvorrichtung (10), umfassend einen Schaft (18), eine Bilderfassungseinrichtung (36), die an einem distalen Endstück (34) des Schafts (18) angeordnet ist und einen Beobachtungsbereich (38) definiert, ein Resektionswerkzeug (20) mit einer Werkzeugspitze (28) und einem Trägerarm (26) und ein Gelenk (110), mittels dessen der Trägerarm (26) gelenkig abwinkelbar ist, wobei in zumindest einer Winkelstellung des Trägerarms (26) ein Abstand (130) zwischen der Werkzeugspitze (28) und einer Längsachse (132) des distalen Endstücks (34) einen Radius (134) eines Querschnittsprofils (136) des distalen Endstücks (34) um wenigstens einen Faktor 2 überschreitet.

## Beschreibung

Die vorliegende Erfindung betrifft Resektoskopvorrichtungen, ein Resektionswerkzeug und ein Resektoskop.

Eine Resektion ist die chirurgische Entfernung von Gewebe eines Organs oder eines Tumors. Je nach Ziel und anatomischen Gegebenheiten können verschiedene Arten von Resektionen durchgeführt werden. Bei schwer zugänglichem Gewebe kann die operative Entfernung endoskopisch mit Hilfe eines Resektoskops erfolgen. Ein solches Resektoskop kommt beispielsweise bei der transurethralen Resektion zum Einsatz, bei der erkranktes Gewebe aus der Harnblase oder von der Prostata entfernt wird. Die Operation erfolgt endoskopisch durch die Harnröhre des Patienten, ohne dass eine Inzision notwendig ist.

Ein herkömmliches Resektoskop für die transurethrale Resektion umfasst einen Schaft, an dessen distalem Endabschnitt eine Beobachtungsoptik, eine Spülvorrichtung und ein Resektionswerkzeug angeordnet sind. Der Schaft ermöglicht den Zugang zur Harnblase durch die Harnröhre und bietet Stabilität während des Eingriffs. Der distale Endabschnitt des Schafts kann daher während des Eingriffs durch Vor- und Zurückschieben des Schafts entlang der Harnröhre sowie durch Schwenken des Schafts in der Harnblase positioniert werden. Bei einem derartigen Schwenken wird ein bezüglich eines Schwenkpunkts proximaler Abschnitt des Schafts von einem Benutzer innerhalb eines großen Manipulationskonus bewegt. Dies ist erforderlich, um entsprechend einen bezüglich des Schwenkpunkts distalen Abschnitt des Schafts in einem hinreichend großen Anwendungskonus bewegen zu können. Ein proximaler Abschnitt des Schafts ist mit einer Bedienbaugruppe gekoppelt, die während des Eingriffs von einem Benutzer gehalten und entsprechend bewegt wird. Die Bedienbaugruppe umfasst zudem eine Betätigungsvorrichtung, mittels derer das Resektionswerkzeug relativ zum distalen Endabschnitt bewegt werden kann.

Eine Resektion mit einem solchen herkömmlichen Resektoskop wird in der Regel wie folgt durchgeführt. Der Benutzer greift das Resektoskop an der Bedienbaugruppe. Dann schiebt er den Schaft derart durch die Harnröhre des Patienten, dass der distale Endabschnitt des Schafts in die Harnblase hineinragt. Anschließend wird die Blase mit Hilfe der Spülvorrichtung gefüllt. Nun wird eine Bildgebungsvorrichtung aktiviert und der Benutzer erhält durch endoskopische Beobachtung einen Überblick über das Innere der Harnblase. Auf diese Weise kann er die zu entfernenden Gewebestellen innerhalb der Harnblase identifizieren. Um diese zu entfernen, schiebt der Benutzer den Schaft vor und schwenkt ihn nach Bedarf, bis sich der distale Endabschnitt in der Nähe der betreffenden Gewebestelle befindet. Er richtet die Beobachtungsoptik der Bildgebungsvorrichtung daraufhin so aus, dass die Gewebestelle scharf im Sichtfeld zu sehen ist. Zum Abtragen des Gewebes betätigt der Anwender nun die Betätigungsvorrichtung, wodurch er das Resektionswerkzeug vor- und zurückschiebt. In einem iterativen Prozess kann dadurch das Gewebe Schicht für Schicht durch Betätigung der Betätigungsvorrichtung abgetragen werden. Um einen größeren Gewebebereich mit dem Resektionswerkzeug zu erreichen, was regelmäßig erforderlich ist, schwenkt und verschiebt der Benutzer den Schaft zusätzlich.

In der beschriebenen Weise kann die transurethrale Resektion vor allem zur Behandlung eines oberflächlichen Blasenkarzinoms zur Resektion in einer Harnblase eingesetzt werden. Die Erfinder der vorliegenden Erfindung haben erkannt, dass dies für den Benutzer schwierig durchzuführen ist und Komplikationen auftreten können. Während der Resektion muss der Benutzer das Resektoskop nach dem Stand der Technik stark schwenken und intensiv entlang der Harnröhre verschieben. Darunter kann eine Präzision des Abtragens des Gewebes leiden. Zudem können Komplikationen für den Patienten auftreten, wobei insbesondere das Schwenken des Schafts häufig zu postoperativen Beschwerden des Patienten führt. Beispielsweise kann es während des Eingriffs zu nicht direkt sichtbaren Verletzungen von Muskeln und Gewebe oder auch zu starken Reizungen der Schleimhäute kommen. Diese Komplikationen können in der Folge zu Harninkontinenz führen. Mit einem beschriebenen herkömmlichen Resektoskop ist, wie erwähnt, stets ein starkes Schwenken des Schafts bei der Durchführung der Resektion erforderlich. Die Resektion wird in Form einer Ruderbewegung durch eine Kombination von Schwenk- und Schubbewegungen durchgeführt. Dadurch wird besonders der Blasenhals großen Belastungen ausgesetzt.

Ausgehend vom Stand der Technik liegt der Erfindung die Aufgabe zugrunde, ein Resektoskop bereitzustellen, mittels dessen eine Resektion präzise und/oder für einen Patienten schonend durchführbar ist.

Die Aufgabe wird erfindungsgemäß durch Resektoskopvorrichtungen, ein Resektionswerkzeug und ein Resektoskop gelöst, wie sie hierin beschrieben und in den Ansprüchen definiert sind.

Gemäß einem Aspekt sieht die vorliegende Erfindung vor, eine Resektoskopvorrichtung, insbesondere zur transurethralen Resektion eines Gewebes, bereitzustellen. Die Resektoskopvorrichtung umfasst einen Schaft und eine Bilderfassungseinrichtung, die an einem distalen Endstück des Schafts angeordnet ist und einen Beobachtungsbereich definiert. Zudem umfasst die Resektoskopvorrichtung ein Resektionswerkzeug mit einer Werkzeugspitze und einem Trägerarm sowie ein Gelenk, mittels dessen der Trägerarm gelenkig abwinkelbar ist. Dabei überschreitet in zumindest einer Winkelstellung des Trägerarms ein Abstand zwischen der Werkzeugspitze und einer Längsachse des distalen Endstücks einen Radius eines Querschnittsprofils des distalen Endstücks um wenigstens einen Faktor 2.

Gemäß einem weiteren Aspekt sieht die vorliegende Erfindung vor, eine Resektoskopvorrichtung, insbesondere zur transurethralen Resektion eines Gewebes, bereitzustellen. Die Resektoskopvorrichtung kann insbesondere oben genannte Merkmale aufweisen. Sie umfasst einen Schaft und eine Bilderfassungseinrichtung, die an einem distalen Endstück des Schafts angeordnet ist und einen Beobachtungsbereich definiert. Zudem umfasst die Resektoskopvorrichtung ein Resektionswerkzeug mit einer Werkzeugspitze und einem Trägerarm sowie ein Gelenk, mittels dessen der Trägerarm gelenkig abwinkelbar ist. Dabei definiert der Schaft einen Manipulationskonus, innerhalb dessen ein bezüglich eines Schwenkpunkts proximaler Abschnitt des Schafts von einem Benutzer manipulierbar ist. Die Werkzeugspitze ist durch Manipulation des proximalen Abschnitts des Schafts innerhalb des Manipulationskonus und Wahl zumindest einer, insbesondere genau einer, Winkelstellung des Trägerarms auf einem innerhalb des Beobachtungsbereichs befindlichen Oberflächenabschnitt eines Anwendungskonus bewegbar. Ein Öffnungswinkel des Anwendungskonus ist wenigstens 1,5-mal so groß wie ein Öffnungswinkel des Manipulationskonus.

Gemäß einem weiteren Aspekt sieht die vorliegende Erfindung vor, eine Resektoskopvorrichtung, insbesondere zur transurethralen Resektion eines Gewebes, bereitzustellen. Die Resektoskopvorrichtung kann insbesondere oben genannte Merkmale aufweisen. Sie umfasst einen Schaft und eine Bilderfassungseinrichtung, die an einem distalen Endstück des Schafts angeordnet ist und einen Beobachtungsbereich definiert. Zudem umfasst die Resektoskopvorrichtung ein Resektionswerkzeug mit einer Werkzeugspitze und einem Trägerarm sowie ein Gelenk, mittels dessen der Trägerarm gelenkig abwinkelbar ist. Dabei umfasst die Bilderfassungseinrichtung zumindest zwei Bilderfassungseinheiten, die in unterschiedliche Blickrichtungen gerichtet sind, wobei die Bilderfassungseinheiten jeweils einen Beobachtungsteilbereich des Beobachtungsbereichs definieren. Die Werkzeugspitze ist durch die Abwinkelung des Trägerarms zwischen den Beobachtungsteilbereichen bewegbar.

Ferner sieht die vorliegende Erfindung vor, ein Resektionswerkzeug, insbesondere für eine erfinderische Resektoskopvorrichtung, bereitzustellen. Es umfasst eine Werkzeugspitze, einen Trägerarm und ein Gelenk, mittels dessen der Trägerarm gelenkig abwinkelbar ist. Das Resektionswerkzeug definiert einen Abwinkelabschnitt, der mittels des Gelenks abwinkelbar ist, wobei die Länge des Abwinkelabschnitts zwischen 15 mm und 50 mm und insbesondere zwischen 25 mm und 35 mm beträgt.

Außerdem sieht die vorliegende Erfindung vor, ein Resektoskop mit einer erfinderischen Resektoskopvorrichtung bereitzustellen.

Die erfindungsgemäßen Merkmale erlauben eine präzise und für einen Patienten schonende transurethrale Resektion. Der Patientenkomfort und die Patientensicherheit werden erhöht sowie das Risiko von Komplikationen wie einer Harninkontinenz reduziert. Dadurch wird in weiterer Folge die Akzeptanz von transurethralen Resektionen erhöht, wodurch Folgen lang aufgeschobener Resektionen vermindert werden. Besonders die nach der herkömmlichen Resektion notwendigen starken Schwenkbewegungen können durch die erfindungsgemäßen Merkmale reduziert werden. Dadurch wird eine grundlegend unterschiedliche Art der Durchführung der Resektion möglich, welche für den Patienten schonend ist und eine hohe Präzision liefert.

Beispielsweise kann ein Benutzer nach dem Einführen des distalen Endstücks einmalig einen Abschnitt des Trägerarms mittels des Gelenks abwinkeln. Dadurch wird die Werkzeugspitze in einen Nahbereich um ein zu entfernendes Gewebe, beispielsweise einen Tumor, geführt. Der Benutzer muss also nicht mehr das gesamte Resektoskop in einem großen Manipulationskonus bewegen bzw. den proximalen Abschnitt des Schafts stark außerhalb der Blase schwenken, um das zu entfernende Gewebe zu erreichen. Die Harnröhre ist hierdurch weniger umfangreichen Bewegungen ausgesetzt. Dies ist konkret dadurch möglich, dass mittels der Abwinkelung des Trägerarms der relativ zum Radius des distalen Endstücks große Abstand der Werkzeugspitze eingestellt werden kann. In dieser einmalig eingestellten Winkelstellung verbleibt die Werkzeugspitze dann zumindest zeitweise während der Durchführung des Gewebeabtrags. Für diesen muss der Benutzer das Resektionswerkzeug und in manchen Fällen das Resektoskop nunmehr vor- und zurückschieben und lediglich kleine Schwenkbewegungen durchführen. Mit einem herkömmlichen Resektoskop müsste der Benutzer, wie oben beschrieben, den Schaft stark schwenken und auf einem Manipulationskonus mit einem großen Öffnungswinkel bewegen. In anderen Worten kann die herkömmliche Ruderbewegung bei der Resektion mittels der erfinderischen Merkmale teilweise oder sogar weitgehend vermieden werden.

Der Anwendungskonus, innerhalb dessen ein Gewebeabtrag möglich ist, hat also einen größeren Öffnungswinkel als der Manipulationskonus. Entsprechend sind beispielsweise Gewebeabschnitte an der unteren oder oberen Blasenwand leicht zu erreichen, obwohl der Manipulationskonus kleingehalten werden kann. Der große Anwendungskonus kann dabei durch die Abwinkelung mittels des Gelenks erreicht werden. Ist die Winkelstellung einmal gewählt, ist die Handhabung des erfinderischen Resektoskops mit herkömmlichen Resektoskopen vergleichbar, sodass Benutzer auf ihre Erfahrung mit herkömmlichen Resektoskopen zurückgreifen können, wenn Eingriffe durchgeführt werden. Der Gewebeabtrag wird durch ein Vor- und Zurückschieben des Resektionswerkzeugs und/oder des Schafts durchgeführt. Im Unterschied sind nunmehr vergleichsweise kleine Schwenkbewegungen des Schafts notwendig.

Ebenfalls denkbar ist eine Ausführungsform, bei der der Gewebeabtrag durch ein kontinuierliches Abwinkeln des mittels des Gelenks abwinkelbaren Abschnitts des Trägerarms relativ zum distalen Endstück durchgeführt wird. Dabei wird die Winkelstellung des Trägerarms kontinuierlich geändert und eine Art Schaufelbewegung durchgeführt. Dadurch wird die Werkzeugspitze entlang des abzutragenden Gewebes geführt und das Gewebe Schicht für Schicht abgetragen. Entsprechend kann zusätzlich das notwendige Vor- und Zurückschieben des Resektionswerkzeugs und/oder des Schafts verringert werden, da durch das Abwinkeln des Trägerarms bereits die Abtragbewegung ausgeführt wird.

Zudem kann durch Verwendung der zumindest zwei Bilderfassungseinheiten ein großer Beobachtungsbereich bereitgestellt werden, der große Teile der Blaseninnenwand abdecken kann (zumindest einseitig). Dieser kann bis zum Blaseneingang bzw. zum Blasenhals reichen. Der Blaseneingang kann also unter allenfalls geringem Schwenken des Schafts im Manipulationskonus eingesehen werden. Da die Werkzeugspitze zwischen den Beobachtungsteilbereichen bewegbar ist, kann diese beim Abwinkeln kontinuierlich mittels des Bilderfassungseinrichtung beobachtet werden und in einen Nahebereich um den Blaseneingang bewegt werden. Es kann also ein großer Abschnitt des Anwendungskonus mittels der Bilderfassungseinrichtung abgedeckt werden, wodurch der Benutzer das distale Endstück nicht stark durch Manipulation des Schafts innerhalb des Manipulationskonus zur endoskopischen Bildgebung schwenken muss. Auch ohne starkes Schwenken kann die Resektion beispielsweise an den Gewebeabschnitten an der unteren oder oberen Blasenwand mittels endoskopischer Bildgebung überwacht werden.

Unter einem "Resektoskop" kann ein medizinisches Instrument zur endoskopischen Entfernung von Gewebe innerhalb eines Hohlorgans, insbesondere der Harnblase und/oder der Prostata, verstanden werden. Ein Resektoskop kann minimalinvasive Eingriffe ermöglichen, insbesondere durch kontrollierte Resektionsbewegungen und/oder endoskopische Bildgebung.

Unter einer "Resektoskopvorrichtung" soll insbesondere ein, vorzugsweise funktionsfähiger Bestandteil, insbesondere eine Unterbaugruppe und/oder eine Konstruktions- und/oder eine Funktionskomponente eines Resektoskops verstanden werden. Vorzugsweise kann die Resektoskopvorrichtung das Resektoskop zumindest teilweise, vorzugsweise zumindest zu einem Großteil und besonders bevorzugt vollständig ausbilden. Beispielsweise kann die Resektoskopvorrichtung dazu eingerichtet sein, zumindest teilweise und vorzugsweise zumindest zu einem Großteil in einen Hohlraum oder in eine insbesondere künstliche und/oder natürliche Kavität, insbesondere Körperkavität, eingeführt zu werden, und zwar insbesondere um diese zu begutachten und Teile davon zu verändern, insbesondere zu entfernen. Bei der Resektoskopvorrichtung kann es sich um eine medizinische Resektoskopvorrichtung handeln.

Unter "eingerichtet" kann im Rahmen dieser Offenbarung insbesondere speziell programmiert, ausgebildet, ausgelegt und/oder ausgestattet verstanden werden. Darunter, dass ein Bauteil zu einer bestimmten Funktion eingerichtet ist, kann im Rahmen dieser Offenbarung insbesondere verstanden werden, dass das Bauteil diese bestimmte Funktion in zumindest einem Anwendungs- und/oder Betriebszustand erfüllt und/oder ausführt.

Unter "distal" soll insbesondere bei einer Bedienung einem Patienten zugewandt und/oder einem Bediener und/oder Benutzer abgewandt verstanden werden. Insbesondere ist proximal das Gegenteil von distal. Unter "proximal" soll insbesondere bei einer Bedienung einem Patienten abgewandt und/oder einem Bediener und/oder Benutzer zugewandt verstanden werden.

Der Schaft kann beispielsweise ein längliches, zylindrisches, insbesondere hohlzylindrisches Element umfassen, das Instrumente, Einrichtungen und/oder dergleichen trägt, mittels derer eine Resektion durchgeführt werden kann. Er kann die Einführung der Instrumente, Einrichtungen und/oder dergleichen in die Kavität, insbesondere die Harnblase, ermöglichen. Allgemein kann mittels des Schafts also ein Zugang zu der Kavität erreicht werden. Dabei kann der Schaft an seinem proximalen Abschnitt von einem Benutzer gehalten und/oder manipuliert werden, um die Instrumente, Einrichtungen und/oder dergleichen innerhalb der Kavität auszurichten. Durch die Manipulation kann insbesondere auch die Resektion durch den Benutzer durchgeführt werden. Der Schaft kann entsprechend starr und/oder biegesteif ausgebildet sein. Der Schaft kann einen Außendurchmesser von beispielsweise bis zu 15 mm, zum Beispiel bis zu 10 mm, insbesondere bis zu 9 mm, bevorzugt bis zu 8 mm, und eine Länge beispielsweise von 10 cm bis zu 50 cm, insbesondere von 15 cm bis zu 40 cm, bevorzugt von 20 cm bis zu 30 cm, aufweisen.

Insbesondere ist der Schaft derart der Kavität des Patienten zuführbar, dass das distale Endstück innerhalb der Kavität angeordnet ist. Das distale Endstück kann Funktionseinheiten der Resektoskopvorrichtung tragen, mittels derer die Resektion durchführbar ist. Das distale Endstück kann einen distalen Endabschnitt des Schafts definieren und sich beispielsweise über bis zu 5 cm, insbesondere bis zu 4 cm, bevorzugt bis zu 3 cm, des Schafts erstrecken.

Unter einem "länglichen Teil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine größte Erstreckung des Bauteils senkrecht zu dessen Haupterstreckung, also insbesondere einem Durchmesser des Bauteils. Unter einer "Haupterstreckung" eines Bauteils, soll insbesondere dessen längste Erstreckung entlang dessen Haupterstreckungsrichtung verstanden werden. Unter einer "Haupterstreckungsrichtung" eines Bauteils soll insbesondere eine Richtung verstanden werden, welche parallel zu einer längsten Kante eines kleinsten gedachten Quaders verläuft, welcher das Bauteil gerade noch vollständig umschließt und welche vorzugsweise durch einen geometrischen Mittelpunkt und/oder durch einen Massenmittelpunkt des Bauteils verläuft.

Mittels der Bilderfassungseinrichtung kann endoskopische Bildgebung durchgeführt werden. Die Bilderfassungseinrichtung kann dazu eingerichtet sein, Objekte innerhalb des Beobachtungsbereichs, insbesondere ein Inneres der Kavität, abzubilden und/oder Bilddaten zu erzeugen. Ein Objekt kann beispielsweise ein Gewebe, insbesondere einen Abschnitt der Blasenwand, umfassen. Anhand der Bilddaten kann an einer Anzeigevorrichtung eine Darstellung des Innern der Kavität erzeugt werden. Über die Anzeigevorrichtung kann der Benutzer also das Innere der Harnblase und einen Fortschritt der Resektion bzw. des Abtragens von Gewebe beobachten. Die Bilderfassungseinrichtung kann, insbesondere durch den Benutzer, innerhalb der Kavität, insbesondere unabhängig von dem Resektionswerkzeug, positionierbar sein.

Die Bilderfassungseinrichtung kann zumindest eine Bilderfassungseinheit umfassen, welche insbesondere durch eine Eingangsoptik und/oder einen Bildsensor zur Bilderzeugung, insbesondere einem CCD-Chip oder CMOS-Chip, ausgebildet sein kann. Die Eingangsoptik kann etwa an einer distalen Endfläche des Schafts angeordnet sein.

Durch die Bilderfassungseinrichtung mit den zumindest zwei Bilderfassungseinheiten, die in unterschiedliche Blickrichtungen gerichtet sind, kann ein großer Beobachtungsbereich für die endoskopische Bildgebung erreicht werden. Jede der Bilderfassungseinheiten kann zumindest durch eine Eingangsoptik und/oder einen Bildsensor zur Bilderzeugung, insbesondere einem CCD-Chip oder CMOS-Chip, ausgebildet sein.

Die wenigstens zwei Bilderfassungseinheiten können insbesondere eine erste Bilderfassungseinheit und eine zweite Bilderfassungseinheit umfassen, wobei die erste Bilderfassungseinheit in eine distale Blickrichtung gerichtet ist, und wobei die zweite Bilderfassungseinheit in eine proximale Blickrichtung gerichtet ist. Die Blickrichtung von zwei verschiedenen Seiten verbessert die dem Benutzer zur Verfügung stehende Abbildung durch die aus verschiedenen Perspektiven aufgenommenen Bilder weiter, und verbessert insbesondere eine Einsehbarkeit beim Setzen eines Ansatzpunktes zu Entfernung von Gewebe.

In einigen Ausführungsformen umfasst die Bilderfassungseinrichtung genau zwei, genau drei und/oder mehrere bzw. zumindest zwei Bilderfassungseinheiten, die in unterschiedliche Blickrichtungen gerichtet sind, wobei die Bilderfassungseinheiten gemeinsam einen durchgängigen Beobachtungsbereich definieren. Die Beobachtungsteilbereiche definieren gemeinsam den Beobachtungsbereich und können sich insbesondere überlappen.

Die Blickrichtung kann bezüglich des Schafts in eine proximale Blickrichtung gerichtet sein. Die Blickrichtung kann bezüglich des Schafts in eine distale Blickrichtung gerichtet sein. Die Blickrichtung kann durch einen Blickwinkel bezüglich der Längsachse des Schafts definiert werden. Ein Blickwinkel von 0° bis weniger als 90° kann als in eine distale Blickrichtung gerichtet bedeuten und ein Blickwinkel von mehr als 90° bis 180° kann als in eine proximale Blickrichtung gerichtet bedeuten.

Die Bilderfassungseinrichtung kann wenigstens drei Bilderfassungseinheiten umfassen, die in drei unterschiedliche Blickrichtungen gerichtet sind. Die Anordnung mehrerer Bilderfassungseinheiten mit unterschiedlichen Blickrichtungen verbessert eine einem Benutzer zur Verfügung stehende Abbildung eines zu entfernenden Gewebes. Die wenigstens drei Bilderfassungseinheiten können drei unterschiedliche Beobachtungsteilbereiche und insbesondere jeweils einen Beobachtungsteilbereich definieren, wobei sich die unterschiedlichen Beobachtungsteilbereiche zumindest teilweise überlappen können. Die Beobachtungsteilbereiche können also jeweils einen Abschnitt aufweisen, der innerhalb eines benachbarten Beobachtungsteilbereichs liegt. Bei überlappenden Beobachtungsteilbereichen, die von verschiedenen Blickrichtungen betrachtet werden, wird die dem Benutzer zur Verfügung stehende Abbildung eines zu entfernenden Gewebes weiter verbessert.

Die überlappenden Beobachtungsteilbereiche können gemeinsam den Beobachtungsbereich definieren. Die mehreren Bilderfassungseinheiten können in einer Längsrichtung und/oder bezüglich einer Längsachse des Schafts, insbesondere des distalen Endstücks des Schafts, versetzt angeordnet sein, insbesondere hintereinander angeordnet sein.

Die eine oder die mehreren Bilderfassungseinheit/en können jeweils durch Kameramodule, insbesondere Kameramodule mit kurzbauenden Objektiven oder sogenannten CameraCubes mit Waferlevel-Optiken gebildet sein. Die eine oder mehrere Bilderfassungseinheit/en weisen insbesondere jeweils einen Einzelsichtwinkel auf. Der Einzelsichtwinkel kann beispielsweise 60°, 65°, 70°, 75°, 80°, 85°, 90°, 95°, 100°, 105° oder 110° betragen.

Die Blickrichtung einer Bilderfassungseinheit kann eine Hauptblickrichtung der jeweiligen Bilderfassungseinheit sein. Beispielsweise kann die Blickrichtung eine Mittelachse einer Eingangsoptik der betreffenden Bilderfassungseinheit sein. Der Einzelsichtwinkel kann ein Winkel sein, der bezüglich der Blickrichtung aufgespannt ist. Insbesondere kann die Mittelachse eine Winkelhalbierende des Einzelsichtwinkels sein. Je nach Ausführung der Bilderfassungseinheiten kann diese durch jeweils zwei Einzelsichtwinkel definiert sein, die senkrecht zueinander aufgespannt sind. Diese können beispielsweise in Sichtwinkel bezüglich einer Querachse bzw. einen Sichtwinkel bezüglich einer Hochachse definieren, die jeweils senkrecht zu der Mittelachse und/oder der Blickrichtung stehen. Insbesondere in Fällen, in denen eine Bilderfassungseinheit einen rechteckigen Bildsensor umfasst, können beide zu der Bilderfassungseinheit gehörenden Sichtwinkel einer kurzen und einer langen Seite des rechteckigen Bildsensors zugeordnet sein.

Die Angabe eines Einzelsichtwinkels kann auch als größter Sichtwinkel der betreffenden Bilderfassungseinheit verstanden werden und/oder als ein Sichtwinkel, der einer Diagonalen des betreffenden Bildsensors entspricht. Eine Bilderfassungseinheit kann auch durch einen horizontalen Sichtwinkel, einen vertikalen Sichtwinkel, und einen diagonalen Sichtwinkel beschreibbar sein. Angaben, die sich auf einen Sichtwinkel beziehen, können sich auf jede dieser drei Größen beziehen und beziehen sich vorzugsweise auf einen diagonalen Sichtwinkel.

Zur Beleuchtung des Innern der Kavität, der Abbildungsbereiche und/oder des Beobachtungsbereichs kann an einem distalen Endstück des Schafts eine Beleuchtungseinrichtung angeordnet sein, die dazu eingerichtet ist, Beleuchtungslicht bereitzustellen. Eine Beleuchtung des Abbildungsbereichs der Abbildungsbereiche und/oder des Beobachtungsbereichs unterstützt die Aufnahme eines Bildes davon mit der Bilderfassungseinrichtung und erhöht somit insbesondere die Bildqualität einer dem Benutzer zur Verfügung gestellten Abbildung. Die Beleuchtungseinrichtung kann mehrere Beleuchtungseinheiten umfassen, die insbesondere in unterschiedliche Beleuchtungsrichtungen gerichtet sind. Die einzelnen Beleuchtungseinheiten können durch Leuchtdioden und/oder Laserdioden und/oder Lichtleiter gebildet sein.

Eine kompakte Resektoskopvorrichtung kann bereitgestellt werden, wenn die Bilderfassungseinrichtung integral mit der Beleuchtungseinrichtung ausgebildet ist. Insbesondere das distale Endstück kann wenig raumgreifend und unter effizienter Bauraumnutzung ausgebildet sein. Die Bilderfassungseinrichtung und die Beleuchtungseinrichtung können in einigen Ausführungsformen paarweise ausgebildet sein. Das kann bedeuten, dass jeweils eine Beleuchtungseinheit integral mit einer Bilderfassungseinheit ausgebildet ist.

Das Resektionswerkzeug kann dazu eingerichtet sein, ein Gewebe mittels Schneidens und/oder mittels Vaporisation zu entfernen. Das Resektionswerkzeug kann ein elektrisch leitfähiges Material, insbesondere einen Draht, über das ein elektrischer Strom fließen kann, aufweisen. Das Resektionswerkzeug kann als HF-Werkzeug (Hochfrequenz-Werkzeug) ausgebildet sein. Das Resektionswerkzeug kann eine Resektionsschlinge aufweisen. Das Resektionswerkzeug kann schlingenförmig, hakenförmig oder kugelförmig sein.

Das Resektionswerkzeug kann insbesondere eine HF-Schlinge (Hochfrequenz-Schlinge) aufweisen. Alternativ oder zusätzlich dazu kann das Resektionswerkzeug einen Laser zum Schneiden und/oder zur Vaporisation aufweisen. Der Laser kann insbesondere zur Holmium-Laser-Enukleation oder zur KTP-Laser-Vaporisation ausgebildet oder als Continuous-Wave-(Dauerstrich)-Laser ausgeführt sein. Diese Laserverfahren unterscheiden sich in der Wellenlänge und Energie des verwendeten Laserlichts, insbesondere also in ihrer Energiewirkung im Gewebe und ihrer Eindringtiefe. Alternativ oder zusätzlich dazu kann das Resektionswerkzeug ein monopolares oder ein bipolares HF-Werkzeug (Hochfrequenz-Werkzeug) aufweisen. Das monopolares HF-Werkzeug kann als Hakenelektrode, Kugelelektrode oder Spatelelektrode zum Schneiden und/oder zur Vaporisation ausgebildet sein. Das bipolare HF-Werkzeug kann insbesondere als eine bipolare Pinzette oder Zange zum Schneiden und/oder zur Vaporisation ausgebildet sein. Das Resektionswerkzeug kann ferner dazu eingerichtet sein, Kontaktkoagulation an einem Gewebe auszuführen. Je nach Anwendungsfall kann eine beschriebene Ausgestaltung des Resektionswerkzeugs besonders geeignet sein.

Dabei kann die Werkzeugspitze den Teil des Resektionswerkzeugs definieren, der mit dem zu entfernenden Gewebe in Kontakt kommt. Darüber hinaus kann die Werkzeugspitze beispielsweise einen Zuleitungsabschnitt und/oder dergleichen umfassen, der sich zumindest von einem distalen Ende des Trägerarms weiter nach distal erstreckt. Insbesondere kann die Werkzeugspitze distal von dem Trägerarm angeordnet sein und/oder sich distal an den Trägerarm anschließen.

Ein Trägerarm kann dazu eingerichtet sein, die Werkzeugspitze mit einer proximalen Baugruppe, insbesondere einer Bedienbaugruppe, zu verbinden. Wenn die Werkzeugspitze elektronisch betrieben ist, kann eine elektrische Zuleitung, beispielsweise ein Kabel, innerhalb des Trägerarms geführt sein und/oder der Trägerarm eine derartige Zuleitung ausbilden.

In einigen Ausführungsformen kann der Trägerarm etwa röhrenförmig ausgebildet sein und zumindest eine weitere funktionelle Einheit und/oder weitere Zuleitungen aufnehmen. Zudem kann ein Trägerarm starr, biegesteif und/oder mechanisch stabil ausgebildet sein. Wird etwa, wie eingangs beschrieben, eine Resektion mit einer einmaligen Wahl einer Winkelstellung des Trägerarms durchgeführt, kann der Trägerarm derart starr, biegesteif und/oder mechanisch stabil ausgebildet sein, dass der Benutzer das zu entfernende Gewebe mit einer für eine Gewebeabtragung eignenden Kraft beaufschlagen kann, ohne dass sich der Trägerarm wesentlich verbiegt.

Mittels des Gelenks ist insbesondere ein distaler Abschnitt des Trägerarms abwinkelbar. Das kann bedeuten, dass sich eine Winkelstellung eines distal vom Gelenk befindlichen Abschnitts relativ zu einem proximal des Gelenks befindlichen Abschnitt verändern lässt. Das Gelenk kann sich insbesondere auf einer Längsachse des Trägerarms befinden und/oder in den Trägerarm integriert sein. In manchen Ausführungsformen bildet der Trägerarm das Gelenk zumindest teilweise aus. Durch das Abwinkeln des Trägerarms kann die Werkzeugspitze innerhalb der Kavität des Patienten positioniert werden, insbesondere ohne eine Manipulation des proximalen Abschnitts des Schafts.

Der abwinkelbare Abschnitt des Trägerarms kann Teil eines "Abwinkelabschnitts" sein. In anderen Worten kann das Resektionswerkzeug den Abwinkelabschnitt definieren, wobei der Abwinkelabschnitt einen Abschnitt des Trägerarms und/oder zumindest teilweise die Werkzeugspitze umfasst. Der Abwinkelabschnitt ist mittels des Gelenks abwinkelbar. Der Abwinkelabschnitt kann die Werkzeugspitze und einen, insbesondere distalen, Abschnitt, insbesondere Endabschnitt, umfassen.

Unter "gelenkig abwinkelbar" kann insbesondere verstanden werden, dass ein Teil des Trägerarms gegenüber einem anderen Teil des Trägerarms gezielt mechanisch verstellbar bzw. drehbar ist. "Gelenkig abwinkeln" kann eine gezielte Bewegung und/oder Ausrichtung umfassen. Ein Bauteil kann als "gelenkig" verstanden werden, wenn es über ein mechanisches Gelenk mit einem anderen Bauteil verbunden ist, insbesondere derart, dass eine relative Bewegung der beiden Bauteile zueinander möglich ist. Dies kann eine einachsige Beweglichkeit, beispielsweise eine Kipp- oder Schwenkbewegung um einen Drehpunkt, umfassen. "Gelenkig abwinkelbar" kann auch eine mehrachsige und/oder multidirektionale Beweglichkeit umfassen, die beispielsweise mittels eines Kugelgelenks ermöglicht wird. Die gelenkige Abwinkelbarkeit kann werkzeugfrei durch Reibung, einen Rastmechanismus und/oder ein Schnappsystem erreicht sein. Zusätzlich kann in einigen Ausführungsformen eine Arretierung vorgesehen sein, mittels derer der abwinkelbare Abschnitt des Trägerarms in einer Abwinkelposition fixiert werden kann.

Der Trägerarm kann insbesondere mehrere Winkelstellungen definieren. In einigen Ausführungsformen kann der Trägerarm mittels des Gelenks kontinuierlich abwinkelbar sein. In anderen Ausführungsformen kann der Trägerarm mittels des Gelenks schrittweise abwinkelbar sein. Ein Winkel der Winkelstellung kann bezüglich einer Längsachse des Schafts, insbesondere des distalen Endstücks und/oder eines, insbesondere unmittelbar, proximal des Gelenks befindlichen Abschnitts des Trägerarms definiert sein. In einer Zuführungskonfiguration, in der die Resektoskopvorrichtung der Kavität zuführbar ist, kann eine Zuführungswinkelstellung definiert sein, wobei der Abwinkelabschnitt und die Längsachse des distalen Endstücks und/oder des, insbesondere unmittelbar, proximal des Gelenks befindlichen Abschnitts des Trägerarms einen Winkel von beispielsweise zwischen einschließlich 5° und -5°, insbesondere zwischen einschließlich 3° und -3°, bevorzugt zwischen einschließlich 1° und -1°, einschließen. Grundsätzlich kann mit der Zuführungswinkelstellung eine Winkelnullstellung gemeint sein, in der der Abwinkelabschnitt parallel zu der Längsachse des distalen Endstücks angeordnet ist. Der Winkel wird beispielsweise ausgehend von der Zuführungswinkelstellung bzw. einer koaxialen Anordnung des Abwinkelabschnitts und einer der o.g. Längsachsen gezählt.

Mit dem Abstand zwischen der Werkzeugspitze und der Längsachse des distalen Endstücks kann insbesondere ein Abstand zwischen einem, insbesondere in der Zuführungswinkelstellung, am weitesten distalen Punkt der Werkzeugspitze gemeint sein. Mit dem Abstand kann ferner eine minimale Distanz zwischen einem am weitesten von der Längsachse entfernten Punkt der Werkzeugspitze und der Längsachse gemeint sein, die insbesondere durch das Abwinkeln mittels des Gelenks einstellbar ist.

Diese minimale Distanz kann den Radius des Querschnittsprofils des distalen Endstücks um wenigstens einen Faktor 2, insbesondere um zumindest einen Faktor 2,5; 3; 3,5; 4; 4,5 und/oder größer als 4,5 überschreiten. In einer Ansicht von distal auf die Resektoskopvorrichtung kann also die Werkzeugspitze bis zu wenigstens diesem Faktor über den Querschnitt des distalen Endstücks hinausragen, insbesondere wenn die Längsachse auf einem geometrischen Mittelpunkt und/oder einem Massenmittelpunkt des Querschnitts des distalen Endstücks ist. Mit dem Radius des Querschnittsprofils kann der größte Radius des Querschnittsprofils gemeint sein. In anderen Worten kann der Radius, der als Bezugsgröße dient, an dem Punkt entlang der Längsachse des distalen Endstücks ermittelt werden, an dem das Querschnittsprofil die größte räumliche, insbesondere quer zur Längsachse, Ausdehnung aufweist. In manchen Ausführungsformen kann der distale Endabschnitt einen kleineren Radius des Querschnittsprofils aufweisen als ein weiter proximaler Abschnitt des Schafts. In einem solchen Fall kann beispielsweise in zumindest einer Winkelstellung des Trägerarms der Abstand zwischen der Werkzeugspitze und der Längsachse des distalen Endstücks einen Radius eines Querschnittsprofils des Schafts um wenigstens einen Faktor 2 überschreiten.

Insbesondere in einer Anwendungskonfiguration überschreitet in zumindest einer Winkelstellung des Trägerarms der Abstand zwischen der Werkzeugspitze und der Längsachse des distalen Endstücks einen Radius eines Querschnittsprofils des distalen Endstücks um wenigstens einen Faktor 2. In der Anwendungskonfiguration ist die Resektion durchführbar. In der Zuführungskonfiguration ist die Resektionsvorrichtung also der Kavität zuführbar und in der Anwendungskonfiguration die Resektion bzw. der Gewebeabtrag durchführbar.

In einem der Kavität zugeführten Zustand kann der Schaft um einen Schwenkpunkt drehbar und/oder linear durch den Schwenkpunkt verschiebbar sein. Unter diesem Schwenkpunkt kann ein sogenannter Trokarpunkt verstanden werden.

Der Schaft erstreckt sich beispielsweise im zugeführten Zustand teilweise außerhalb der Kavität und teilweise innerhalb der Kavität. Insbesondere der proximale Abschnitt des Schafts befindet sich außerhalb der Kavität und zumindest das distale Endstück innerhalb der Kavität.

Der Schaft, insbesondere der proximale Abschnitt des Schafts, kann durch den Benutzer um den Schwenkpunkt bzw. den Trokarpunkt in einem konusförmigen Bereich bewegt werden. Der Schwenkpunkt bzw. der Trokarpunkt beschreibt dabei die Spitze des Konus. Die Bewegung bzw. die Manipulation kann ein Schwenken und ein Vor- und Zurückschieben des Schafts umfassen.

Für jeden Öffnungswinkel, der durch ein Schwenken des proximalen Abschnitts des Schafts gewählt wird, kann der Benutzer also eine Mantelfläche eines gedachten, extrakavitären Konus abfahren. Dieser kann entsprechend als Manipulationskonus definiert sein. Der gedachte, extrakavitäre Konus kann insbesondere die Form eines geraden Kreiskegels aufweisen.

Ein einzelner Manipulationskonus weist also einen bestimmten Öffnungswinkel auf. Der Benutzer kann den proximalen Schaftabschnitt innerhalb des Manipulationskonus durch ein Schwenken und ein lineares Verschieben durch den Schwenkpunkt manipulieren, wobei der Öffnungswinkel nicht überschritten wird.

Das distale Endstück und der proximale Abschnitt des Schafts können sich beide hintereinander auf der Längsachse des Schafts befinden. Entsprechend beschreibt das distale Endstück eine Bewegung innerhalb eines Bewegungskonus, wenn der Benutzer den proximalen Abschnitt des Schafts innerhalb des Manipulationskonus manipuliert. Der Bewegungskonus weist dann einen Öffnungswinkel auf, der dem Öffnungswinkel des Manipulationskonus entspricht. Entsprechend kann der Bewegungskonus einer Spiegelung des Manipulationskonus entsprechen. In der Zuführungskonfiguration kann also die Werkzeugspitze im Wesentlichen innerhalb des Bewegungskonus durch Manipulation des proximalen Abschnitts des Schafts bewegbar sein.

Zusätzlich kann der Benutzer den Trägerarm mittels des Gelenks abwinkeln. Genauer kann er den Abwinkelabschnitt abwinkeln. Dadurch kann er eine Winkelstellung des Trägerarms wählen. In der Winkelstellung kann die Werkzeugspitze einen Abstand zu der Längsachse des distalen Abschnitts aufweisen. Insbesondere in der Anwendungskonfiguration ist der Trägerarm abgewinkelt und/oder kann eine Winkelstellung, insbesondere ungleich der Winkelstellung der Zuführungskonfiguration, aufweisen.

In der Anwendungskonfiguration kann die Werkzeugspitze, insbesondere aufgrund der Distanz der Werkzeugspitze zur Längsachse des distalen Endstücks, außerhalb des Bewegungskonus durch Manipulation des proximalen Abschnitts des Schafts bewegbar sein.

Im abgewinkelten Zustand und/oder in der Anwendungskonfiguration ist die Werkzeugspitze durch die Manipulation des proximalen Abschnitts des Schafts innerhalb des Manipulationskonus auf dem Anwendungskonus bewegbar. Ein bestimmter Manipulationskonus erlaubt dabei eine Bewegung der Werkzeugspitze auf einem bestimmten Anwendungskonus.

Da der Trägerarm abgewinkelt sein kann und insbesondere die Werkzeugspitze einen Abstand zu der Längsachse des distalen Endstücks aufweisen kann, kann der Anwendungskonus einen größeren Öffnungswinkel aufweisen als der Bewegungskonus und entsprechend der Manipulationskonus. Wird der proximale Abschnitt des Schafts innerhalb eines bestimmten Manipulationskonus bewegt, kann die Werkzeugspitze auf der Oberfläche des Anwendungskonus bewegbar sein. Dabei kann mit der Werkzeugspitze ein Oberflächenabschnitt des Anwendungskonus abfahrbar sein, der beispielsweise einer Mantelfläche eines Kegelstumpfs entsprechen kann. Der Schwenkpunkt selbst kann von der Oberfläche des Anwendungskonus, die mit der Werkzeugspitze durch Manipulation des proximalen Abschnitts des Schafts abfahrbar ist, ausgenommen sein.

Um die Werkzeugspitze auf der Mantelfläche des Kegelstumpfs und/oder dem Oberflächenabschnitt des Anwendungskonus zu bewegen, kann der proximale Abschnitt des Schafts innerhalb des Manipulationskonus näher zu der Achse des Manipulationskonus bewegt werden. Die Oberfläche des Anwendungskonus kann also durch eine lineare Bewegung durch den Schwenkpunkt und eine Schwenkbewegung um den Schwenkpunkt des proximalen Abschnitts des Schafts innerhalb des Manipulationskonus mittels der Werkzeugspitze abfahrbar sein. Entsprechend kann die Werkzeugspitze innerhalb des Anwendungskonus bewegbar sein durch eine Manipulation des proximalen Abschnitts des Schafts innerhalb des Manipulationskonus, wobei der Anwendungskonus einen größeren Öffnungswinkel aufweist als der Manipulationskonus.

Der Anwendungskonus kann allgemein die Grenze eines Bereichs angeben, innerhalb dessen eine Resektion unter Beibehaltung eines bestimmten Manipulationskonus durchführbar ist. Aufgrund der Abwinkelbarkeit des Trägerarms kann der Manipulationskonus einen vergleichsweise kleinen Öffnungswinkel aufweisen. Dadurch kann eine für den Patienten schonende Resektion durchgeführt werden. Diese kann auch für den Benutzer komfortabel durchführbar sein, da er einfach und effizient abgelegene und/oder entfernte Gewebebereiche innerhalb der Harnblase erreichen kann.

In anderen Worten kann aufgrund der Wahl der Winkelstellung bei gleich starker Schwenkung um den Schwenkpunkt des Schafts ein größerer Bereich mittels der Werkzeugspitze erreicht werden im Vergleich zu einer herkömmlichen Resektoskopvorrichtung, die kein Gelenk bzw. kein Gelenk mit den erfinderischen Merkmalen aufweist. Insbesondere ist der Trägerarm zumindest derart mittels des Gelenks abwinkelbar, dass der Öffnungswinkel des Anwendungskonus wenigstens 1,5-mal, insbesondere 2,0-mal, 2,5-mal, 3,0-mal, 3,5-mal, 4,0-mal, und/oder 4,5-mal so groß ist wie der Öffnungswinkel des Manipulationskonus.

Der Öffnungswinkel des Manipulationskonus muss beispielsweise gemäß einigen Ausführungsformen einen Öffnungswinkel von 15°, insbesondere 10°, nicht übersteigen, um zumindest im Wesentlichen die gesamte Blasenwand mittels der Resektionsspitze erreichen zu können. Der notwendige Öffnungswinkel kann von der Anatomie des Patienten sowie der Länge des Abwinkelabschnitts abhängen. Wenn die Länge des Abwinkelabschnitts zwischen 15 mm und 50 mm und insbesondere zwischen 25 mm und 35 mm beträgt, kann mit einem kleinen Öffnungswinkel des Manipulationskonus zumindest im Wesentlichen die gesamte Blasenwand mit der Werkzeugspitze für einen Gewebeabtrag erreicht werden. Denkbar ist, dass Werkzeugspitze und/oder das Resektionswerkzeug patientenspezifisch gewählt wird/werden, um die Länge des Abwinkelabschnitts mit der Anatomie des Patienten abzustimmen.

Insbesondere kann sich der abfahrbare Bereich der Werkzeugspitze mit dem Beobachtungsbereich schneiden. Der überlappende Teil kann als der Oberflächenabschnitt des Anwendungskonus bezeichnet werden, auf dem die Werkzeugspitze bewegbar ist. Dieser befindet sich also innerhalb des Beobachtungsbereichs. Entsprechend kann die Werkzeugspitze mittels der endoskopischen Bildgebung beim Abfahren des Oberflächenabschnitts des Anwendungskonus beobachtbar sein. Der Oberflächenabschnitt muss nicht zwangsweise ausschließlich durch den Beobachtungsbereich begrenzt sein. Eine Grenze, insbesondere eine Grenze proximal zum Schwenkpunkt, kann durch die Wahl der Winkelstellung definiert sein. Eine distale Grenze kann durch den Beobachtungsbereich begrenzt sein.

Gemäß einigen Ausführungsformen definiert das Resektionswerkzeug den Abwinkelabschnitt, der mittels des Gelenks abwinkelbar ist. Ein Abstand des Oberflächenabschnitts des Anwendungskonus kann zum Schwenkpunkt wenigstens 2-mal so groß sein wie die Länge des Abwinkelabschnitts, insbesondere bei der Wahl genau einer Winkelstellung. Es lassen sich also auch weit vom Schwenkpunkt entfernte Gewebeabschnitte der Kavität, insbesondere der Harnblase, mit der Werkzeugspitze erreichen. Die geometrischen Zusammenhänge können sich grundsätzlich auf eine bestimmte Winkelstellung beziehen. Erreicht werden können beispielsweise Gewebeabschnitte, die zumindest 10 mm, insbesondere zumindest 20 mm, bevorzugt zumindest 30 mm, besonders bevorzugt zumindest 40 mm, von dem Blaseneingang entfernt liegen. Mit dem Abstand kann jeweils ein minimaler Abstand gemeint sein, also insbesondere ein Abstand zwischen einem dem Gewebeabschnitt nächsten Punkt des Blaseneingangs und einem dem Blaseneingang nächsten Punkt des Gewebeabschnitts.

Grundsätzlich kann die Länge des Abwinkelabschnitts eine Länge der Werkzeugspitze und eine Länge eines mittels des Gelenks abwinkelbaren Abschnitts des Trägerarms umfassen.

Unter "zwischen den Beobachtungsteilbereichen bewegbar sein" kann auch verstanden werden, dass die Werkzeugspitze von einem Beobachtungsteilbereich in einen anderen Beobachtungsteilbereich durch die Abwinkelung des Trägerarms bewegbar ist. Alternativ könnte also beispielsweise formuliert werden, dass die Werkzeugspitze durch die Abwinkelung des Trägerarms von einem ersten Beobachtungsteilbereich einer ersten Bilderfassungseinheit in einen zweiten Beobachtungsteilbereich einer zweiten Bilderfassungseinheit bewegbar ist. Insbesondere ist die Werkzeugspitze zwischen den Beobachtungsteilbereichen hin und her bewegbar. Insbesondere ist die Werkzeugspitze aus einem der Beobachtungsteilbereiche, insbesondere dem ersten Beobachtungsteilbereich, durch die Abwinkelung des Trägerarms herausbewegbar.

Ferner kann der Trägerarm mittels des Gelenks relativ zum distalen Endstück des Schafts abwinkelbar sein, wobei insbesondere das distale Endstück relativ zu einem Grundkörper des Schafts unbeweglich ist. Dadurch wird eine schonende Resektion ermöglicht, bei der der Benutzer den Schaft allenfalls geringfügig schwenken muss. Da die Bilderfassungseinrichtung am distalen Endstück angeordnet ist, verbleibt diese beim Abwinkeln des Trägerarms ortsfest. Beim Überführen von der Zuführungskonfiguration in die Anwendungskonfiguration muss entsprechend die Bilderfassungseinrichtung nicht bewegt werden. Der Trägerarm kann unabhängig von der Bilderfassungseinrichtung und/oder dem distalen Endstück abwinkelbar sein. Der Grundkörper kann ein Abschnitt des Schafts sein, der sich im in die Harnblase eingeführten Zustand innerhalb des Harnleiters befindet. Ferner kann der Grundkörper beispielsweise den proximalen Abschnitt des Schafts umfassen. Er kann etwa ein strukturelles Basiselement des Schafts bilden, das insbesondere die grundlegende Form und Stabilität des Schafts bereitstellt. Der Grundkörper kann also das zentrale Trägerelement darstellen, an dem sich zumindest das distale Endstück anschließt. Das distale Endstück kann biegesteif, starr und/oder unbeweglich mit dem Grundkörper verbunden sein.

Eine schonende Resektion kann durchgeführt werden, wenn der Schaft und das Resektionswerkzeug unabhängig voneinander entlang einer Längsachse des Schafts bewegbar sind. Für einen Gewebeabtrag muss also nicht die gesamte Resektoskopvorrichtung vor und zurück bewegt werden. Beispielsweise kann das Resektionswerkzeug mit einer proximalen Bedienbaugruppe gekoppelt sein, die, wenn sie durch den Benutzer bedient wird, das Resektionswerkzeug unabhängig vom Schaft entlang der Längsachse des Schafts bewegt.

Zudem kann das Resektionswerkzeug den Abwinkelabschnitt definieren, der mittels des Gelenks abwinkelbar ist, wobei die Länge des Abwinkelabschnitts den Radius des Querschnittsprofils des distalen Endstücks um wenigstens einen Faktor 2, insbesondere um wenigstens einen Faktor 3, überschreitet. Zum Verständnis und den Vorteilen dieser Merkmale wird auf obige Ausführungen verwiesen.

Außerdem kann die Länge des Abwinkelabschnitts zwischen 15 mm und 50 mm und insbesondere zwischen 25 mm und 35 mm betragen. Zum Verständnis und den Vorteilen dieser Merkmale wird auf obige Ausführungen verwiesen.

Gemäß einigen Ausführungsformen umfasst die Resektoskopvorrichtung ferner eine Spülvorrichtung, mittels derer ein durch das Resektionswerkzeug bearbeitbarer Bereich spülbar ist. Die Spülvorrichtung umfasst eine Zuleitung, mittels derer eine Spülflüssigkeit entlang des Schafts leitbar ist, wobei sich die Zuleitung innerhalb des Trägerarms erstreckt. Während der Abtragung von Gewebe kann abgetragenes Gewebe weggespült werden, sodass eine gute Sicht auf das zu bearbeitende Gewebe gewährleistet werden kann. Die Spülvorrichtung kann einen Kanal umfassen, der in den Trägerarm eingebracht ist und/oder sich entlang einer Längsachse des Trägerarms erstreckt. Der Kanal kann die Zuleitung ausbilden. Alternativ kann die Zuleitung einen Schlauch umfassen, der sich innerhalb eines Hohlraums des Trägerarms entlang der Längsachse von proximal nach distal erstreckt. Proximal kann dadurch Spülflüssigkeit in die Zuleitung eingeleitet und entlang des Trägerarms in die Kavität geleitet werden. Mittels der Spülflüssigkeit kann die Harnblase zudem gefüllt werden, um die Blasenwand zu spannen. Insbesondere in einem bei einer Resektion in die Harnblase bzw. die Kavität eingebrachten distalen Abschnitt des Trägerarms kann die Zuleitung innerhalb des Trägerarms angeordnet sein.

Ferner kann die Spülvorrichtung einen Auslauf umfassen, der an einem mittels des Gelenks abwinkelbaren Abschnitt des Trägerarms angeordnet ist. In anderen Worten kann der Abwinkelabschnitt den Auslauf umfassen und/oder der Auslauf an einem Abschnitt des Abwinkelabschnitt angeordnet sein, der den Trägerarm umfasst. Der Auslauf kann also durch Betätigung des Gelenks abgewinkelt werden. Bei einer Abwinkelung des Trägerarms können sich der Auslauf und die Werkzeugspitze gemeinsam bewegen. Der Auslauf der Spülvorrichtung kann also eine feste Relativposition bezüglich der Werkzeugspitze aufweisen. Die Spülung und/oder das Entfernen von abgetragenen Geweberesten kann dadurch effizient durchgeführt werden. Die Spülflüssigkeit kann unabhängig von der Winkelstellung zielgenau bereitgestellt werden.

Gemäß einigen Ausführungsformen umfasst das Resektionswerkzeug einen weiteren abwinkelbaren Trägerarm. Zwischen dem Trägerarm und dem weiteren Trägerarm kann die Werkzeugspitze angeordnet sein. Insbesondere der Schaft erstreckt sich zumindest in einer Zuführungskonfiguration zwischen den abwinkelbaren Abschnitten der Trägerarme. Dadurch kann eine platzsparende, kompakte Resektoskopvorrichtung kann bereitgestellt werden. Die Werkzeugspitze kann insbesondere eine HF-Resektionsschlinge umfassen. In jedem der Trägerarme kann jeweils eine Zuleitung angeordnet sein, wobei jede der Zuleitungen jeweils einen Pol darstellen kann.

Zu entfernende Gewebeabschnitte können sich auch in Längsrichtung des Schafts an der Blasenwand bzw. gegenüber dem Blaseneingang befinden. Um diese einfach und unter einer geringen Schwenkung des proximalen Abschnitts des Schafts erreichen zu können, kann die Werkzeugspitze eine HS-Resektionsschlinge umfassen, die einen Zuleitungsabschnitt und einen Arbeitsabschnitt umfasst, wobei der Arbeitsabschnitt relativ zu einer Längsachse des Zuleitungsabschnitts um einen Schlingenwinkel abgewinkelt ist, der höchstens 90° beträgt. Insbesondere erstreckt sich der Arbeitsabschnitt teilweise distal von dem Zuleitungsabschnitt. Herkömmliche Resektoskope mit einer HS-Resektionsschlinge weisen in der Regel Schlingenwinkel größer als 90° auf. Diese müssen entsprechend stark geschwenkt werden, damit der Arbeitsabschnitt das zu entfernende Gewebe gegenüber dem Blaseneingang erreichen kann. Der Arbeitsabschnitt kann der Abschnitt der Werkzeugspitze sein, der mit dem zu entfernenden Gewebe für den Gewebeabtrag in Kontakt kommt. Der Zuleitungsabschnitt kann eine Zuleitung umfassen und/oder eine Zuleitung den zumindest teilweise Zuleitungsabschnitt ausbilden. In manchen Ausführungsformen weist der Zuleitungsabschnitt eine elektrische Isolierung auf. Ein Schlingenwinkel von 0° kann bedeuten, dass der Arbeitsabschnitt koaxial mit dem Zuleitungsabschnitt angeordnet ist und/oder sich distal von dem Zuleitungsabschnitt auf der Längsachse des Zuleitungsabschnitts erstreckt. Beispielsweise bei einem Schlingenwinkel von 45° erstreckt sich der Arbeitsabschnitt schräg von dem Zuleitungsabschnitt teilweise nach distal und teilweise senkrecht zur Längsachse des Zuleitungsabschnitts.

Eine umfassende endoskopische Bildgebung der Harnblase kann erreicht werden, wenn das distale Endstück des Schafts derart für die Durchführung einer Resektion zumindest teilweise innerhalb einer Harnblase anordenbar ist, dass sich der Blasenhals innerhalb des Beobachtungsbereich der Bilderfassungseinrichtung befindet. Selbst komplexe Resektionen nahe des Blasenhalses können unter einer geringen Schwenkung des Resektoskops durchgeführt werden, wobei die Werkzeugspitze im Beobachtungsbereich gehalten werden kann. Entsprechend obigen Ausführungen bzgl. der Bilderfassungseinrichtung, auf die verwiesen wird, kann die Bilderfassungseinrichtung zumindest zwei Bilderfassungseinheiten umfassen, die in unterschiedliche Blickrichtungen gerichtet sind, wobei die Bilderfassungseinheiten jeweils einen Beobachtungsteilbereich des Beobachtungsbereichs definieren. Eine der Blickrichtungen kann derart angeordnet sein, dass der Beobachtungsteilbereich der zugeordneten Bilderfassungseinheit den Schaft schneidet.

Gewebe der gesamten Blasenwand kann einfach mit der Werkzeugspitze erreicht werden, wenn das Resektionswerkzeug gemeinsam mit dem Schaft um eine Längsachse des Schafts drehbar ist. Die Bilderfassungseinrichtung kann also gemeinsam mit der Werkzeugspitze gedreht werden und beispielsweise die Blasendecke mit der Werkzeugspitze erreicht werden, wobei sie weiterhin mittels der endoskopischen Bildgebung beobachtet werden kann.

Die Resektoskopvorrichtung kann ferner eine Einführhülse umfassen, die einer Kavität eines Patienten teilweise zuführbar ist und eine Längsachse definiert. Der Schaft kann sich innerhalb der Einführhülse erstrecken und der Schaft und das Resektionswerkzeug unabhängig voneinander relativ zu der Einführhülse entlang der Längsachse der Einführhülse bewegbar sein. Insbesondere sind der Schaft und das Resektionswerkzeug gemeinsam mit der Einführhülse der Kavität zuführbar. Durch die Einführhülse kann eine Gewebeschädigung und/oder -reizung durch die lineare Bewegung des Schafts durch den Schwenkpunkt reduziert werden.

Die Einführhülse kann als ein längliches Rohr ausgebildet sein und/oder ein solches umfassen, entlang dessen Längsachse insbesondere ein Führungskanal verlaufen kann. Insbesondere kann sie dazu eingerichtet sein, einen Zugang zu der Harnblase eines Patienten bereitzustellen. Dazu kann sie in einer Harnröhre des Patienten anordenbar und/oder in diese einführbar sein. In dieser angeordnet kann sich die Längsachse und insbesondere der Führungskanal entlang der Harnröhre erstrecken. In diesem Fall erstreckt sich die Einführhülse von einer proximalen Seite zu einer distalen Seite. In dem Führungskanal können der Schaft und/oder das Resektionswerkzeug zumindest teilweise und/oder abschnittsweise geführt sein und insbesondere geführt vorschiebbar sein. In einigen Ausführungsformen kann der Schaft in der Einführhülse und das Resektionswerkzeug an dem Schaft geführt sein. Die Einführhülse kann also den Schaft und das Resektionswerkzeug in sich aufnehmen, insbesondere an einem Abschnitt, der innerhalb der Harnröhre anordenbar ist. Ebenso können andere Baugruppen in der Einführhülse aufgenommen sein, beispielsweise ein Absaugkanal zum Absaugen von Spülflüssigkeit aus der Harnblase. Die Einführhülse kann an dem Schaft verliersicher angebracht sein. In anderen Worten kann die Einführhülse relativ zum Schaft bewegbar und am Schaft befestigt sein. Eine intuitive Bedienbarkeit kann insbesondere dann erzielt werden, wenn der Schaft relativ zu der Einführhülse einen linearen Bewegungsfreiheitsgrad und einen Rotationsfreiheitsgrad aufweist, im Speziellen eine axiale Bewegbarkeit in Kombination mit einer Drehbarkeit aber einer Ortsfestigkeit bezüglich einer radialen Position. In einigen Ausführungsformen kann eine Drehposition fest und/oder festlegbar sein. Die Resektoskopvorrichtung kann dazu einen durch den Benutzer bedienbaren Arretiermechanismus umfassen. Im arretierten Zustand kann dann lediglich die axiale Bewegbarkeit verbleiben. Alternativ oder zusätzlich kann auch eine axiale Position arretierbar sein. Hierfür kann ebenfalls ein Arretiermechanismus vorgesehen sein, insbesondere der genannte Arretiermechanismus. In diesem Fall kann im arretierten Zustand lediglich die Drehbarkeit als Bewegungsfreiheitsgrad verbleiben. Eine besonders intuitive Bedienbarkeit wird für den Fall erzielt, dass der Schaft relativ zur Einführhülse linear beweglich und drehbar ist.

In anderen Worten kann die Einführhülse eine Funktion in der Art eines Trokars, im Speziellen eines verliersicher angebrachten Trokars, der aus der minimal-invasiven Chirurgie bekannt ist, aufweisen. Sie kann also trokarartig einen Zugang zu der Kavität, insbesondere der Harnblase, bereitstellen.

Die Längsachse kann parallel zur Haupterstreckungsrichtung der Einführhülse verlaufen. In einem in die Kavität zumindest teilweise zugeführten Zustand kann eine Längsachse des Schafts ebenso parallel zur Haupterstreckungsrichtung der Einführhülse und/oder deren Längsachse verlaufen.

Die Einführhülse kann eine kleinere Längserstreckung als der Schaft aufweisen. In einigen Ausführungsformen kann die Längserstreckung der Einführhülse beispielsweise höchstens 95 %, insbesondere höchstens 80 %, bevorzugt höchstens 70 %, besonders bevorzugt höchstens 50 %, der Längserstreckung des Schafts betragen. Mit der Längserstreckung ist die Erstreckung entlang der Längsachse gemeint. Der Schaft kann beispielsweise zwischen 20 cm und 40 cm, insbesondere zwischen 20 cm und 30 cm, bevorzugt zwischen 20 cm und 25 cm lang sein.

Die Einführhülse kann einen ringförmigen, insbesondere kreisringförmigen, Querschnitt, insbesondere in einem distalen Endstück, aufweisen. Die Wanddicke der Einführhülse kann beispielsweise 0,5 mm bis 1,5 mm betragen. Ein Außendurchmesser kann beispielsweise bis zu 10 mm, insbesondere bis zu 9 mm, bevorzugt bis zu 8 mm betragen. Grundsätzlich ist dabei ein kleiner Durchmesser wünschenswert, um eine Belastung für den Patienten kleinzuhalten.

Die Einführhülse kann aus Edelstahl, einem medizinischen Kunststoff, Titan, Polyetheretherketon und/oder dergleichen gefertigt sein. In manchen Ausführungsformen ist die Einführhülse für eine Einmalverwendung vorgesehen, bzw. ist ein Single-Use-Produkt. An seiner Innenfläche kann die Einführhülse eine reibungsreduzierende Beschichtung aufweisen, beispielsweise aus Teflon, Silikon und/oder dergleichen.

Die Resektoskopvorrichtung kann zudem eine proximale Bedienbaugruppe umfassen, die durch einen Benutzer greifbar ist und mittels derer der Schaft manipulierbar ist. Der Benutzer kann die Werkzeugspitze also durch ein Manipulieren der Bedienbaugruppe innerhalb der Harnblase ausrichten.

Ferner kann die Bedienbaugruppe als Ganzes relativ zu der Einführhülse bewegbar sein. Die Einführhülse kann also bei einer Bewegung der proximalen Bedienbaugruppe ruhen und/oder sich lediglich minimal bewegen. Ist die Einführhülse entsprechend einer Harnröhre zugeführt, kann dadurch eine Bewegung der Einführhülse relativ zu der Harnröhre reduziert werden, wobei eine longitudinale Beweglichkeit des Schafts und/oder des Resektionswerkzeugs nicht eingeschränkt ist. Der Schaft und/oder das Resektionswerkzeug können beispielsweise proximal mit der Bedienbaugruppe gekoppelt sein. Durch ein Vor- und Zurückbewegen der Bedienbaugruppe kann der Benutzer dadurch den Schaft und das Resektionswerkzeug, insbesondere gemeinsam, relativ zu der Einführhülse bewegen und insbesondere eine Abtragungsbewegung durchführen.

Der Schaft und das Resektionswerkzeug können also insbesondere von einem Benutzer unabhängig voneinander bewegt werden, wobei die Bewegung relativ zur Einführhülse durchgeführt wird. Für eine Resektion kann der Benutzer die Einführhülse gemeinsam mit dem Schaft und dem Resektionswerkzeug der Harnröhre zuführen. Dies kann auch nacheinander geschehen, beispielsweise, wenn die verschiedenen Baugruppen unterschiedlich lang sind. Unter "gemeinsam" kann entsprechend verstanden werden, dass das Zuführen ein einzelner Vorgang sein kann und der Benutzer die einzelnen Baugruppen nicht nacheinander greifen muss und einzeln durch die Harnröhre vorschieben muss. In einem in der Harnröhre bzw. der Kavität teilweise zugeführten Zustand kann der Benutzer wahlweise das Resektionswerkzeug und/oder den Schaft weiter vorschieben, insbesondere um die Harnblase zu erkunden und/oder eine Resektion durchzuführen.

Bezugnehmend auf die obigen Ausführungen bzgl. der unabhängigen Bewegbarkeit des Resektionswerkzeugs relativ zum Schaft umfasst die proximale Bedienbaugruppe in einigen Ausführungsformen ferner einen Betätigungsmechanismus, mittels dessen das Resektionswerkzeug entlang der Längsachse unabhängig von dem Schaft bewegbar ist. Durch eine Betätigung des Betätigungsmechanismus kann der Benutzer das Resektionswerkzeug, insbesondere unabhängig vom Schaft und/oder der Einführhülse, vor- und zurückschieben, insbesondere um eine Abtragungsbewegung durchzuführen.

In anderen Worten können der Schaft und das Resektionswerkzeug dazu eingerichtet sein, nach einem Zuführen in die Kavität bei einem Abtragen von Gewebe innerhalb der Einführhülse bewegt zu werden. Beim Abtragen von Gewebe wird also im Unterschied zu einer herkömmlichen Resektion nicht mehr eine Baugruppe, welche in Kontakt mit der Harnröhre steht, verschoben.

Die Einführhülse kann lösbar mit der Bedienbaugruppe koppelbar sein. Beispielsweise kann die Bedienbaugruppe gemeinsam mit der Einführhülse einen Koppelmechanismus ausbilden, der dazu eingerichtet ist, die Einführhülse lösbar mit der Bedienbaugruppe zu verbinden. Bedarfsweise kann die Einführhülse dadurch freigegeben werden, beispielsweise nach einem mit dem Schaft und dem Resektionswerkzeug gemeinsamen Einbringen in die Kavität. Ebenfalls kann die Einführhülse bedarfsweise wieder an die Bedienbaugruppe mechanisch gekoppelt werden, um die Einführhülse gemeinsam mit dem Schaft und dem Resektionswerkzeug aus der Kavität zu entfernen.

Gemäß den obigen Ausführungen kann die Resektoskopvorrichtung verschiedene Stellungen aufweisen, die durch eine unterschiedliche Längsstellung des Resektionswerkzeugs zu dem Schaft und zu der Einführhülse und/oder eine unterschiedliche Längsstellung des Schafts zu dem Resektionswerkzeug und zu der Einführhülse definiert sein können.

Wie bereits beschrieben, kann die Einführhülse dazu eingerichtet sein, bei einem Abtragen von Gewebe atraumatisch zu ruhen. Der Patientenkomfort wird erhöht und postoperative Komplikationen werden reduziert. Es versteht sich, dass sich die Einführhülse geringfügig relativ zum Harnleiter bewegen könnte. Diese Bewegung kann jedoch vernachlässigbar klein sein, bzw. deutlich kleiner als eine Relativbewegung zwischen einem Schaft und der Harnröhre bei einer herkömmlichen Resektion. In anderen Worten kann die Einführhülse dazu eingerichtet sein, bei einem Positionieren des Schafts innerhalb der Kavität in Bezug auf ein sie umgebendes und insbesondere sie kontaktierendes Gewebe zumindest im Wesentlichen zu ruhen.

Außerdem kann die Resektoskopvorrichtung einen Rücklauf für eine Spülflüssigkeit umfassen, wobei der Rücklauf zwischen der Einführhülse und dem Schaft ausgebildet ist. Geweberückstände, die bei der Abtragung entstehen, können aus der Harnblase ausgespült werden, um eine Trübung der Flüssigkeit, insbesondere der Spülflüssigkeit, in der Harnblase zu reduzieren und eine gute Sicht auf das zu entfernende Gewebe zu gewährleisten. In einigen Ausführungsformen kann ein Spiel zwischen der Einführhülse und dem Schaft vorgesehen sein, wodurch ein Spalt entstehen kann. Dieser kann den Rücklauf ausbilden. Der Außendurchmesser des Schafts kann etwa bis zu 10 %, insbesondere bis zu 7 %, bevorzugt bis zu 4 %, kleiner sein als ein Innendurchmesser der Einführhülse. Die Resektoskopvorrichtung kann proximal an eine Absaugeinrichtung angeschlossen sein, mittels derer ein Absaugdruck erzeugbar ist, um die Spülflüssigkeit aus der Kavität, insbesondere der Harnblase, heraufzubefördern.

Eine präzise Abwinkelung und Positionierung der Werkzeugspitze können erreicht werden, wenn das Gelenk ein mechanisches Drehgelenk ist. Dadurch kann also eine verbesserte Steuerbarkeit und höhere Stabilität der Resektoskopvorrichtung erreicht werden. Unter einem mechanischen Drehgelenk kann eine Verbindungsvorrichtung zwischen zwei Abschnitten des Trägerarms verstanden werden, die diese derart verbindet, dass sie relativ zueinander um eine feste Drehachse rotieren können.

Das Gelenk kann ferner, insbesondere mittels der Bedienbaugruppe, durch einen Benutzer betätigbar sein. Das kann bedeuten, dass der Benutzer durch eine Betätigung den Abwinkelabschnitt abwinkeln kann. Beispielsweise könnte die Resektoskopvorrichtung einen Betätigungsmechanismus zur Betätigung des Gelenks umfassen, beispielsweise einen Bowdenzug, einen Zug- und/oder Druckmechanismus, ein Steuerkabel, eine Druckstange und/oder einen Zugdraht. Denkbar ist auch ein Betätigungsmechanismus umfassend eine Zahnstange und ein Ritzel, wobei beispielsweise das Ritzel an dem Abwinkelabschnitt angeordnet sein kann. Durch eine Linearbewegung der Zahnstange entlang der Längsachse des Trägerarms kann entsprechend der Abwinkelabschnitt abwinkelbar sein. Der Betätigungsmechanismus kann sich zumindest teilweise innerhalb des Trägerarms erstrecken, beispielsweise innerhalb des Hohlraums des Trägerarms.

Eine besonders präzise Positionierung der Werkzeugspitze kann erreicht werden, wenn das Gelenk einen einzelnen Freiheitsgrad aufweist. Mittels des Gelenks kann der Abwinkelabschnitt beispielsweise um eine Drehachse drehbar sein, die senkrecht zu und/oder durch die Längsachse des Trägerarms verläuft. In anderen Worten kann die Drehachse des Gelenks quer zu der Längsrichtung des distalen Endstücks verlaufen. In einigen Ausführungsformen umfasst die Resektoskopvorrichtung zwei Trägerarme, wobei die Drehachse in diesen Ausführungsformen senkrecht zu der jeweiligen Längsachse der beiden Trägerarme verläuft. Insbesondere ist die Drehachse derart angeordnet, dass die Werkzeugspitze innerhalb des Beobachtungsbereichs abwinkelbar ist.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung einer Resektoskopvorrichtung in einer Seitenansicht;
- Fig. 2: eine schematische Darstellung der Resektoskopvorrichtung in einer Ansicht von distal;
- Fig. 3: eine perspektivische schematische Darstellung der Resektoskopvorrichtung in einer ersten Stellung;
- Fig. 4: eine perspektivische schematische Darstellung der Resektoskopvorrichtung in einer zweiten Stellung;
- Fig. 5.: eine schematische Seitenansicht einer Resektoskopvorrichtung in einer dritten Stellung;
- Fig. 6: eine schematische Darstellung eines Resektoskops umfassend die Resektoskopvorrichtung, einer Steuereinheit und einer Anzeigeeinrichtung;
- Fig. 7: eine schematische Darstellung der Resektoskopvorrichtung in einer Seitenansicht;
- Fig. 8: eine schematische Darstellung eines Teilquerschnitts der Resektoskopvorrichtung;
- Fig. 9: eine weitere schematische Darstellung der Resektoskopvorrichtung;
- Fig. 10: eine weitere schematische Darstellung der Resektoskopvorrichtung;
- Fig. 11: eine schematische Darstellung eines distalen Abschnitts der Resektoskopvorrichtung in einer Seitenansicht;
- Fig. 12: eine weitere schematische Darstellung der Resektoskopvorrichtung;
- Fig. 13: eine weitere schematische Darstellung der Resektoskopvorrichtung;
- Fig. 14: eine weitere schematische Darstellung der Resektoskopvorrichtung;
- Fig. 15: eine weitere schematische Darstellung der Resektoskopvorrichtung;
- Fig. 16: eine weitere schematische Darstellung der Resektoskopvorrichtung;
- Fig. 17: eine weitere schematische Darstellung der Resektoskopvorrichtung; und
- Fig. 18: eine weitere schematische Darstellung der Resektoskopvorrichtung.

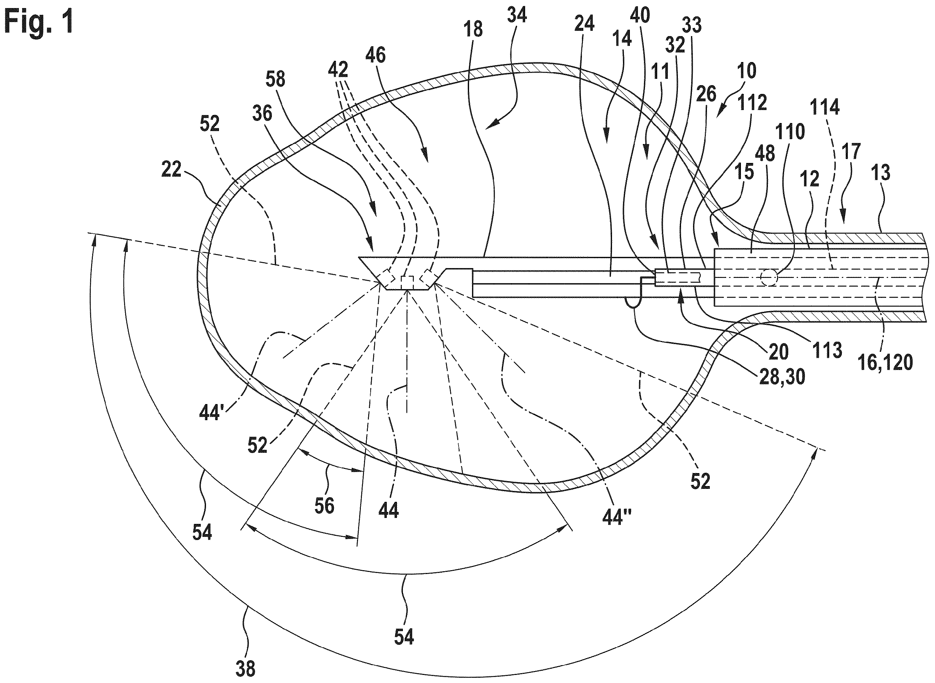

Die Fig. 1 zeigt eine schematische Darstellung einer Resektoskopvorrichtung 10 in einer Seitenansicht. Die Resektoskopvorrichtung 10 ist zur Durchführung einer Resektion eingerichtet und umfasst eine Einführhülse 12, die eine Längsachse 16 aufweist, einen Schaft 18 und ein Resektionswerkzeug 20. Die Resektoskopvorrichtung 10 ist in einer von vielen möglichen Stellungen dargestellt. Die Stellungen werden durch eine Relativposition der Einführhülse 12, des Schafts 18 und des Resektionswerkzeugs 20 zueinander definiert. Diese Baugruppen 12, 18, 20 sind relativ zueinander unabhängig voneinander bewegbar.

Die Einführhülse 12 ist in einem in eine Harnröhre 13 eingeführten Zustand darstellt. Ein distales Endstück 15 der Einführhülse 12 ragt in eine Harnblase 11 hinein. Denkbar ist auch, dass in anderen Situationen, beispielsweise abhängig von einer Anatomie eines Patienten, die Einführhülse 12 nicht bis in die Harnblase 11 hineinragt. Da die Einführhülse 12 röhrenförmig ausgebildet ist und einen Führungskanal 17 definiert, stellt sie trokarartig einen Zugang zu der Harnblase 11 für den Schaft 18 und das Resektionswerkzeug 20 zur Verfügung.

Der Schaft 18 und das Resektionswerkzeug 20 erstrecken sich entlang der Längsachse 16 der Einführhülse 12 durch den Führungskanal 17 hindurch in die Kavität 14, in diesem Fall die Harnblase 11. Diese Baugruppen 18, 20 sind gemeinsam mit der Einführhülse 12 in die Harnröhre 13 einbringbar, indem sie mechanisch miteinander gekoppelt sind. Sobald die Einführhülse 12 in einer gewünschten Position angeordnet ist, können der Schaft 18 und das Resektionswerkzeug 20 weiter nach distal vorgeschoben werden.

Der Schaft 18 weist ein distales Endstück 34 auf, das unbeweglich mit einem Grundkörper 19 des Schafts 18 verbunden ist. An dem distalen Endstück 34 weist der Schaft 18 eine Bilderfassungseinrichtung 36 für eine endoskopische Bildgebung des Innern der Harnblase 11 und eine Beleuchtungseinrichtung 46 für eine Beleuchtung des Innern der Harnblase 11 auf. Die Bilderfassungseinrichtung 36 und die Beleuchtungseinrichtung 46 sind integral miteinander ausgebildet.

Die Bilderfassungseinrichtung 36 umfasst drei Bilderfassungseinheiten 42, die unterschiedliche Blickrichtungen 44, 44', 44" definieren. Die Bilderfassungseinheiten 42 weisen darüber hinaus jeweils einen Sichtwinkel 52 auf. Eine Bilderfassungseinheit 42 weist eine Blickrichtung 44' nach distal und eine andere Bilderfassungseinheit 42 eine Blickrichtung 44" nach proximal auf. Die Bilderfassungseinheiten 42 definieren gemeinsam einen durchgängigen Beobachtungsbereich 38. Jede der Bilderfassungseinheiten 42 weist entsprechend dem jeweiligen Sichtwinkel 52 einen eigenen Beobachtungsteilbereich 54 auf. Diese weisen jeweils zumindest einen Überlappungsbereich 56 auf, der einen Beobachtungsteilbereich 54 einer benachbarten Bilderfassungseinheit teilweise überlappt. Die Bilderfassungseinheiten 42 umfassen jeweils eine Eingangsoptik und einen Bildsensor zur Bilderzeugung (beide nicht im Detail dargestellt), der insbesondere einem CCD-Chip oder CMOS-Chip umfasst. Die Bilderfassungseinheiten 42 sind integral mit Beleuchtungseinheiten (nicht im Detail dargestellt) der Beleuchtungseinrichtung 46 ausgebildet, welche als LEDs ausgebildet sind.

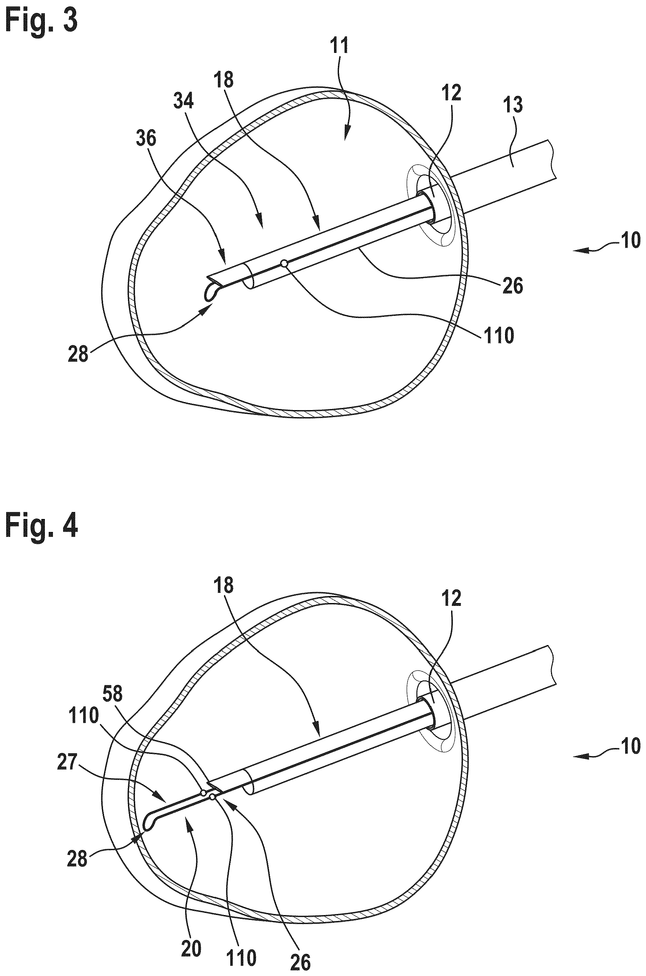

Das Resektionswerkzeug 20 umfasst zumindest einen Trägerarm 26 und eine Werkzeugspitze 28, die als HF-Resektionsschlinge ausgebildet ist. Genauer umfasst das Resektionswerkzeug 20 zwei Trägerarme 26, wie im Folgenden noch beschrieben wird. An einer Oberfläche des Schafts 18 ist eine Führungseinrichtung 24 ausgebildet, die als Führungsnut für den Trägerarm 26 ausgeführt ist. Die Führungseinrichtung 24 erstreckt sich entlang eines Großteils des Schafts 18 bis in das distale Endstück 34 hinein. Das Ende der Führungseinrichtung 24 im distalen Endstück 34 ist als offene Führungsnut vorgesehen. Die Führungseinrichtung 24, insbesondere die Führungsnut, nimmt Trägerarme 26 des Resektionswerkzeugs 20 teilweise in sich auf. Dadurch sind die Trägerarme 26 in der Führungseinrichtung 24 linear entlang einer Längsachse 120 des Schafts 18 geführt. Die Längsachse 120 ist koaxial mit der Längsachse 16 angeordnet. Der Schaft 18 und das Resektionswerkzeug 20 sind unabhängig voneinander entlang der Längsachse 120 des Schafts 18 bewegbar. Die Trägerarme 26 können also in der Führungseinrichtung 24 linear von proximal nach distal und umgekehrt verschoben werden. Durch diese Verschiebung kann die Werkzeugspitze 28 linear relativ zum Schaft 18 und unabhängig von diesem verfahren werden. Insbesondere kann die Werkzeugspitze 28 über ein distales Ende 58 des Schafts 18 hinaus verfahren werden (siehe Fig. 4).

Weiterhin umfasst das Resektionswerkzeug 20, insbesondere der Trägerarm 26, ein Gelenk 110, mittels dessen der Trägerarm 26 gelenkig abwinkelbar ist. Der Trägerarm 26 bildet das Gelenk 110 zumindest teilweise aus. Genauer ist mittels des Gelenks 110 ein Abwinkelabschnitt 112 relativ zu einem bezüglich des Gelenks 110 proximalen Abschnitt 114 des Trägerarms 26 gelenkig abwinkelbar. Der Abwinkelabschnitt 112 umfasst einen abwinkelbaren Abschnitt 113 des Trägerarms 26 und die Werkzeugspitze 28. Entsprechend ist der Trägerarm 26, insbesondere der Abwinkelabschnitt 112, mittels des Gelenks 110 relativ zum distalen Endstück 34 des Schafts 18 abwinkelbar. Dadurch ist die Werkzeugspitze 28 innerhalb des Beobachtungsbereichs 38 bewegbar. Zum Gelenk 110 und dessen Funktion wird zudem auf die nachfolgenden Figuren verwiesen.

Außerdem umfasst die Resektoskopvorrichtung 20 eine Spülvorrichtung 32. Diese umfasst eine Zuleitung 33 und einen Auslauf 40. Die Zuleitung 33 erstreckt sich entlang einer Längsachse des Trägerarms 18 und innerhalb des Trägerarms 26 von proximal nach distal über das Gelenk 110 hinweg bis zu dem Auslauf 40. Der Auslauf 40 ist an dem Trägerarm 26 angeordnet, insbesondere an einem distalen Ende des Trägerarms 26, insbesondere an dem abwinkelbaren Abschnitt 113 des Trägerarms 26. Durch die Zuleitung 33 ist eine Spülflüssigkeit führbar, die am Auslauf 40 austritt. Mittels der Spülflüssigkeit kann die Harnblase 11 für eine Resektion gefüllt werden und zudem Geweberückstände, die bei der Resektion anfallen, weggespült werden.

Die Zuleitung 33 kann abschnittsweise als Kanal ausgebildet sein, der in dem Trägerarm 26 ausgebildet ist. Abschnittsweise kann die Zuleitung 33 zudem durch eine flexible Leitung wie einen Schlauch ausgebildet sein (nicht im Detail dargestellt). Mittels der flexiblen Leitung kann die Spülflüssigkeit über das Gelenk 110 leitbar sein. In einigen Ausführungsformen ist die flexible Leitung in dem Hohlraum (nicht dargestellt) des Trägerarms 26 über zumindest einen Großteil der Längserstreckung des Trägerarms 26 geführt.

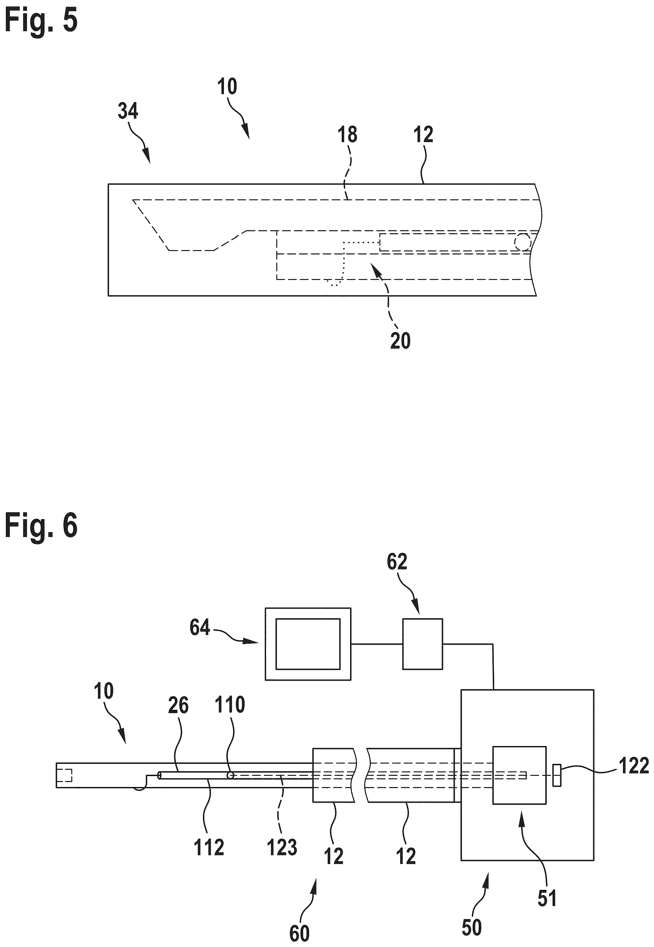

Außerdem umfasst die Resektoskopvorrichtung 10 eine proximale Bedienbaugruppe (siehe Fig. 6), die als Ganzes relativ zu der Einführhülse 12 bewegbar ist. Ein Benutzer kann die Resektoskopvorrichtung 10 an der Bedienbaugruppe halten und ein Resektoskop umfassend die Resektoskopvorrichtung 10 (siehe Fig. 6) bedienen. Die proximale Bedienbaugruppe ist durch den Benutzer greifbar und durch sie ist der Schaft 18 manipulierbar.

Mittels der Resektoskopvorrichtung 10 kann wie folgt eine Resektion durch den Benutzer durchgeführt werden. Zunächst führt er den Schaft 18 und das Resektionswerkzeug 20 gemeinsam mit der Einführhülse 12 der Kavität 14, vorliegend die Harnröhre 13 und teilweise die Harnblase 11, zu. Dabei bringt er die Einführhülse 12 in eine gewünschte Position innerhalb der Harnröhre 13. Nun füllt er die Harnblase 11 unter Verwendung der Spülvorrichtung 32, um diese aufzuspannen. Anschließend positioniert er das distale Endstück 34 des Schafts 18 umfassend die Bilderfassungseinrichtung 36 innerhalb der Harnblase 11. Dazu schiebt er den Schaft 18 relativ zu der Einführhülse 12 nach distal vor. Die Einführhülse 12 verbleibt in der ursprünglichen Position. Dadurch wird eine gesamte zurückgelegte Strecke einer Relativbewegung zwischen einer Resektoskopvorrichtung und der Harnröhre im Vergleich zum Stand der Technik reduziert.

Bei dem Vorschieben ist das Resektionswerkzeug 20 mit dem Schaft 18 gekoppelt. Das heißt, beide Baugruppen 18, 20 werden gemeinsam vorgeschoben. Durch ein Schwenken des Schafts 18 und ein bedarfsweises Vorschieben kann sich der Benutzer einen Überblick über das Innere der Harnblase 11 verschaffen und das Gewebe 22 an einer Innenwand der Harnblase 11 begutachten. Dadurch kann er abzutragendes Gewebe identifizieren, welches etwa eine pathologische Veränderung zeigt. Ein Beispiel für ein solches Gewebe ist etwa kanzeröses Gewebe.

Dieses identifizierte Gewebe kann daraufhin mittels der Werkzeugspitze 28 abgetragen werden. Dazu bringt der Benutzer die Werkzeugspitze 28 durch Abwinkeln des Abwinkelabschnitts 112, Schwenken des Schafts 18 und longitudinales Positionieren des Schafts 18 in einen Nahbereich um das Gewebe. Dabei positioniert er das distale Endstück 34 derart, dass das abzutragende Gewebe im Beobachtungsbereich 38 angeordnet ist und dieses gut abgebildet werden kann. Beim Iongitudinalen Positionieren verbleibt die Einführhülse 12, wie bereits beschrieben, ortsfest relativ zu der Harnröhre 13 bzw. der Kavität 14. In anderen Worten ruht die Einführhülse 12 atraumatisch.

Nach dem Positionieren des distalen Endstücks 34 und einer Wahl einer Winkelstellung des Abwinkelabschnitts 112 (siehe folgende Figuren) verfährt der Benutzer das Resektionswerkzeug 20 gemäß einer Ausführungsform relativ zu dem Schaft 18, wobei die Einführhülse 12 ebenfalls ortsfest, atraumatisch ruht, und bringt die Werkzeugspitze 28 in Kontakt mit dem Gewebe. Nach Aktivierung der Werkzeugspitze 28, wenn diese etwa als HF-Resektionsschlinge ausgebildet ist, kann das Gewebe schichtweise abgetragen werden. Dazu wird die Werkzeugspitze 28 iterativ über das Gewebe gestrichen und dabei oberflächlich das abzutragende Gewebe bearbeitet und abgetragen. Zum Überstreichen des Gewebes kann einerseits das Resektionswerkzeug 20 relativ zum Schaft 18 bewegt werden. Darüber hinaus kann der Schaft 18 gemeinsam mit dem Resektionswerkzeug 20 bewegt werden, wobei jedoch die Einführhülse 12 ortsfest in der Harnröhre 13 verbleibt. Während der Bewegung wird die Winkelstellung beibehalten.

Gemäß einer anderen Ausführungsform wird die Gewebeabtragungsbewegung durch das Abwinkeln des Abwinkelabschnitts 112 durchgeführt. Der Schaft 18 kann dabei nahezu ortsfest gehalten werden. Für nähere Ausführungen zu beiden Ausführungsformen wird auf die folgenden Figuren verwiesen.

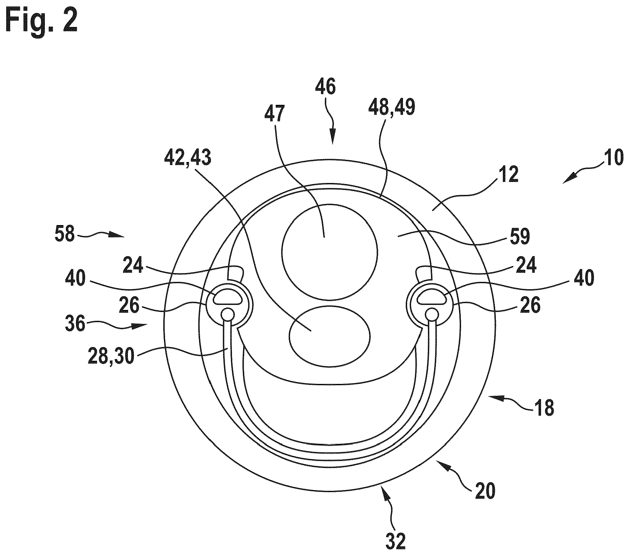

Während des Abtragens des Gewebes wird konstant mittels der Spülvorrichtung 32 gespült und Spülflüssigkeit aus der Harnblase 11 mittels eines Rücklaufs 48 (siehe Fig. 2) aus der Harnblase 11 herausbefördert.

Nach erfolgter Resektion kann der Schaft 18 gemeinsam mit dem Resektionswerkzeug 20 und der Einführhülse 12 aus der Kavität 14 herausgezogen werden.

Der Schaft 18 und das Resektionswerkzeug 20 sind also dazu eingerichtet, nach einem Zuführen in die Kavität 14 bei dem Abtragen von Gewebe 22 innerhalb der Einführhülse 12 bewegt zu werden. Dabei ist die Einführhülse 12 dazu eingerichtet, bei dem Abtragen von Gewebe 22 atraumatisch zu ruhen.

Fig. 2 zeigt eine schematische Darstellung der Resektoskopvorrichtung 10 in einer Ansicht von distal auf ein distales Ende 58 des Schafts 18 bzw. auf eine distale Endfläche der Resektoskopvorrichtung 10. Man erkennt eine distale Endfläche 59 des Schafts 18, an der eine Beleuchtungseinheit 47 der Beleuchtungseinrichtung 56 angeordnet ist, die als LED ausgebildet ist. Ebenfalls ist an der distalen Endfläche 59 eine Eingangsoptik 43 einer der Bilderfassungseinheiten 42 angeordnet.

Zudem erkennt man die Führungseinrichtung 24, deren distales Ende seitlich in die distale Endfläche 59 eingelassen ist. In der Führungseinrichtung 24 sind die Trägerarme 26 geführt. Das Resektionswerkzeug 20 umfasst zwei Trägerarme 26. Zwischen den Trägerarmen 26 ist die Werkzeugspitze 28, insbesondere die HF-Resektionsschlinge 30, angeordnet. Der Schaft 18 ist zwischen den Trägerarmen 26 angeordnet. Ferner erkennt man den Auslauf 40 der Spülvorrichtung 32 des Trägerarms 26. Beide Trägerarme 26 sind baugleich ausgebildet und weisen entsprechend jeweils einen Auslauf 40 auf.

Der Schaft 18 hat in der Einführhülse 12 Spiel. Daher ist ein Spalt 49 zwischen der Einführhülse 12 und dem Schaft 18 zu sehen. Dieser Spalt 49 bildet den Rücklauf 48 aus. Die Spülflüssigkeit kann also durch den Spalt 49 abgesaugt werden.

Die Einführhülse 12 weist in dem gezeigten Ausführungsbeispiel einen Außendurchmesser von 9 mm und einen Innendurchmesser von 7.5 mm auf. Der Schaft 18 weist einen Außendurchmesser von 6.5 mm auf. Der Schaft 18 weist eine Länge von 30 cm auf. Die Einführhülse 12 weist eine Länge von 25 cm auf. Denkbar sind auch deutlich kürzere Einführhülsen 12, die beispielsweise halb so lang sind wie der Schaft 18. Noch kürzere Einführhülsen 12 sind auch denkbar. Die genannten Maße sind lediglich beispielhaft zu verstehen.

Die Fig. 3 und die Fig. 4 zeigen jeweils eine perspektivische schematische Darstellung der Resektoskopvorrichtung 10 in unterschiedlichen Stellungen. Diese sind durch eine unterschiedliche Relativposition des Resektionswerkzeugs 20 bezüglich dem Schaft 18 definiert.

In der Fig. 3 ist die Resektionsvorrichtung 10 mit der Bilderfassungseinrichtung 36 im distalen Endstück 34 des Schafts 18 zu sehen. Ferner sieht man das Resektionswerkzeug 20 mit der Werkzeugspitze 28 und einem der Trägerarme 26 sowie dem Gelenk 110. Außerdem sieht man die Einführhülse 12, die der Harnröhre 13 zugeführt ist. Der Schaft 18 und das Resektionswerkzeug 20 erstrecken sich durch die Einführhülse 12 hindurch und in die Harnblase 11 hinein. Der Schaft 18 und das Resektionswerkzeug 20 können gemeinsam relativ zu der Einführhülse 12 bewegt werden, wobei die Einführhülse 12 ortsfest in der Harnröhre 13 verbleibt. In der in der Fig. 3 gezeigten Stellung ist die Werkzeugspitze 28 bündig mit dem distalen Ende 58 des Schafts 18 angeordnet.

Wie in der Fig. 4 zu sehen, kann das Resektionswerkzeug 20 unabhängig von dem Schaft 18 relativ zu diesem und zur Einführhülse 12 bewegt werden. In der in der Fig. 4 gezeigten Stellung ist die Werkzeugspitze 28 distal über das distale Ende 58 des Schafts 18 hinaus verfahren angeordnet. Die Einführhülse 12 ist in derselben Position wie gemäß der Fig. 3 verblieben. Das Resektionswerkzeug 20 wurde also unabhängig von dem Schaft 18 bewegt. Zudem erkennt man einen weiteren Trägerarm 27. Dieser ist entsprechend den obigen Ausführungen baugleich mit dem Trägerarm 26 ausgebildet. Er ist entsprechend ebenfalls abwinkelbar. Ferner erkennt man jeweils ein Gelenk 110 je Trägerarm 26, 27. Zwischen dem Trägerarm 26 und dem weiteren Trägerarm 27 ist die Werkzeugspitze 28 angeordnet. Bezugnehmend auf die vorigen Figuren erstreckt sich der Schaft 118 in der jeweils gezeigten Winkelstellung zwischen den abwinkelbaren Abschnitten 113 der Trägerarme 26, 27. Diese Konfiguration wird als Zuführungskonfiguration bezeichnet. Da die Trägerarme 26, 27 nicht abgewinkelt sind, kann das Resektionswerkzeug 20 der Körperhöhle über die Einführhülse 12 zugeführt werden. Entsprechend ist die gezeigte Winkelstellung eine Zuführungswinkelstellung. In dieser erstreckt sich der Abwinkelabschnitt 112 parallel zu den Längsachsen 16, 120.

Fig. 5 zeigt eine schematische Seitenansicht der Resektoskopvorrichtung 10 in einer weiteren Stellung. In der gezeigten Stellung ist das distale Endstück 34 des Schafts 18 vollständig von der Einführhülse 12 eingefasst. Das Resektionswerkzeug 20 ragt ebenfalls nicht distal über die Einführhülse 12 hinaus. In der gezeigten Stellung könnte die Resektoskopvorrichtung 10 beispielsweise der Kavität 14 zugeführt werden. Ebenso denkbar sind allerdings auch andere Stellungen, in denen zumindest eine der Baugruppen 18, 20 distale über die Einführhülse 12 hinausragt.

Fig. 6 zeigt eine schematische Darstellung des Resektoskops 60, das die Resektoskopvorrichtung 10 umfasst. Ferner erkennt man die proximale Bedienbaugruppe 50, die als Ganzes relativ zu der Einführhülse 12 bewegbar ist. Die proximale Bedienbaugruppe 50 umfasst zudem eine Betätigungsvorrichtung 51, mittels derer das Resektionswerkzeug 20 bedienbar, insbesondere vor- und zurückschiebbar ist. Ferner bildet die Bedienbaugruppe 50 zumindest teilweise eine Betätigungsvorrichtung 122 zur Betätigung des Gelenks 110 aus. Die Betätigungsvorrichtung ist nicht näher gezeigt. Denkbar ist beispielsweise die Verwendung einer Zahnstange, die sich etwa entlang der Linie 123 innerhalb des Trägerarms 26 zum Gelenk 110 erstreckt. An dem Abwinkelabschnitt 112 kann entsprechend ein Ritzel ausgebildet sein, das in die Zahnstange eingreift. Der Benutzer kann also durch Betätigung der Zahnstange den Abwinkelabschnitt 112 abwinkeln.

Das Resektoskop 60 ist mit einer Steuereinheit 62 verbunden, die beispielsweise Bilddaten verarbeiten kann und eine Funktion des Resektoskops 60 steuern kann. Insbesondere kann die Bilderfassungseinrichtung mittels der Steuereinheit 62 gesteuert werden. Die Steuereinheit 62 ist mit einer Anzeigeeinrichtung 64 verbunden, an der eine Darstellung eines endoskopischen Bilds erzeugt werden kann. Über die Darstellung kann der Benutzer das Innere der Harnblase 11 betrachten und so auch die Durchführung der Resektion überwachen.

Anhand der folgenden Figuren werden eine Funktionsweise und die Merkmale der Resektoskopvorrichtung 10 näher erläutert.

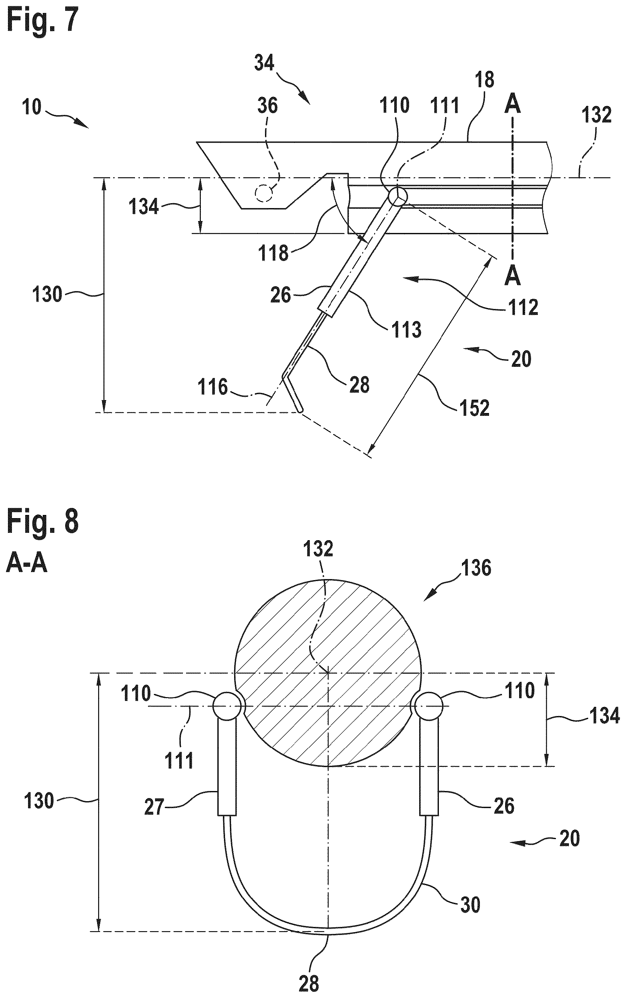

Fig. 7 und Fig. 8 zeigen verschiedene schematische Darstellungen der Resektoskopvorrichtung 10. Fig. 7 zeigt sie in einer Seitenansicht und Fig. 8 einen Teilquerschnitt der Resektoskopvorrichtung 10. Die Resektoskopvorrichtung 10 umfasst den Schaft 18, die Bilderfassungseinrichtung 36 (siehe Fig. 1 bis Fig. 6), die an dem distalen Endstück 34 des Schafts 18 angeordnet ist, und das Resektionswerkzeug 20. Dieses weist die Werkzeugspitze 28, den Trägerarm 26 und das Gelenk 110 auf. Mittels des Gelenk 110 ist das Resektionswerkzeug 20 abschnittsweise abwinkelbar. Konkret ist der Abwinkelabschnitt 112 bezüglich des proximal des Gelenks 110 angeordneten Abschnitts des Trägerarms 26 abwinkelbar und durch das Abwinkeln in eine Winkelstellung versetzbar. Der Abwinkelabschnitt 112 umfasst, wie bereits ausgeführt, den abwinkelbaren Abschnitt 113 des Trägerarms 26 und die Werkzeugspitze 28. Wie in Fig. 8 zu erkennen ist, umfasst die Resektoskopvorrichtung zwei Trägerarme 26, 27, zwischen denen sich die Werkzeugspitze 28 erstreckt.

In Fig. 7 und Fig. 8 ist das Resektionswerkzeug 20 in jeweils in einer Winkelstellung dargestellt. In dieser ist der Abwinkelabschnitt 112 bezüglich der Längsachse 132 des distalen Endstücks 34 abgewinkelt. Der Trägerarm 26, insbesondere der abwinkelbare Abschnitt 113 des Trägerarms 26, ist also relativ zum distalen Endstück 34 des Schafts 18 abwinkelbar. In der dargestellten Winkelstellung des Trägerarms 26 gemäß Fig. 7 hat die Werkzeugspitze 28 einen Abstand 130 zu der Längsachse 132 des distalen Endstücks 34. Der Abstand 130 beschreibt die Distanz zwischen dem am weitesten von der Längsachse 132 entfernten Punkt des Abwinkelabschnitts 112 und der Längsachse 132. Dieser Abstand 130 ist um etwa einen Faktor 4 größer als ein Radius 134 eines Querschnittsprofils 136 des distalen Endstücks 34 (siehe auch Fig. 8). Der Radius 134 ist im dargestellten Fall ein größter Radius des distalen Endstücks 34, wobei ein während der Resektion innerhalb der Körperhöhle bzw. der Harnblase anordenbarer Abschnitt des Schafts 18 das distale Endstück 34 definiert. In der Fig. 8 ist eine Winkelstellung dargestellt, bei der der Abstand 130 doppelt so groß ist wie der Radius 134.

Ferner erkennt man in Fig. 7 eine Länge 152 des Abwinkelabschnitts 112. Diese ist als eine Distanz zwischen dem Gelenk 110 und einem am weitesten vom Gelenk 110 entfernten Punkt des Abwinkelabschnitts 112 gemessen, wobei die Distanz parallel zur Haupterstreckungsrichtung des abwinkelbaren Abschnitts 113 gemessen wird. Die Länge 152 des Abwinkelabschnitts 112 überschreitet den Radius 134 des Querschnittsprofils 136 des distalen Endstücks 34 etwa um einen Faktor 3,5. Genauer beträgt die Länge 152 des Abwinkelabschnitts 112 25 mm.

Das Gelenk 110 ist ein mechanisches Drehgelenk und weist einen einzelnen Freiheitsgrad auf. Dieser ist ein Rotationsfreiheitsgrad. Eine Drehachse 111 des Gelenks 110 verläuft orthogonal zur Längsachse 132 des distalen Endstücks 34 (siehe Fig. 7 und Fig. 8). Wie in der Fig. 8 zu sehen, verläuft die Drehachse 111 auf einer gedachten Verbindungslinie zwischen den jeweiligen Gelenken 110 der Trägerarme 26, 27. Die Trägerarme 26, 27 weisen also jeweils ein Gelenk 110 auf, das jeweils eine Drehachse 111 definiert. Die Drehachsen 111 der Gelenke 110 sind koaxial angeordnet.

Mittels des Gelenks 110 ist der abwinkelbare Abschnitt 113 in eine Winkelstellung verfahrbar, in der eine Haupterstreckungsachse des abwinkelbaren Abschnitts 113 und die Längsachse 132 einen Armwinkel 118 von wenigstens 45° einschließen. In manchen Ausführungsformen erlaubt das Gelenk einen Armwinkel 118 von zumindest 90°, insbesondere zumindest 135°, und/oder einen Armwinkel 118 von bis zu beispielsweise 170°, insbesondere 175°.

Wie im Folgenden noch genauer ausgeführt wird, definieren die in Fig. 7 und Fig. 8 Winkelstellungen des Trägerarms 26 jeweils eine Anwendungskonfiguration, in der eine Resektion durchgeführt wird.

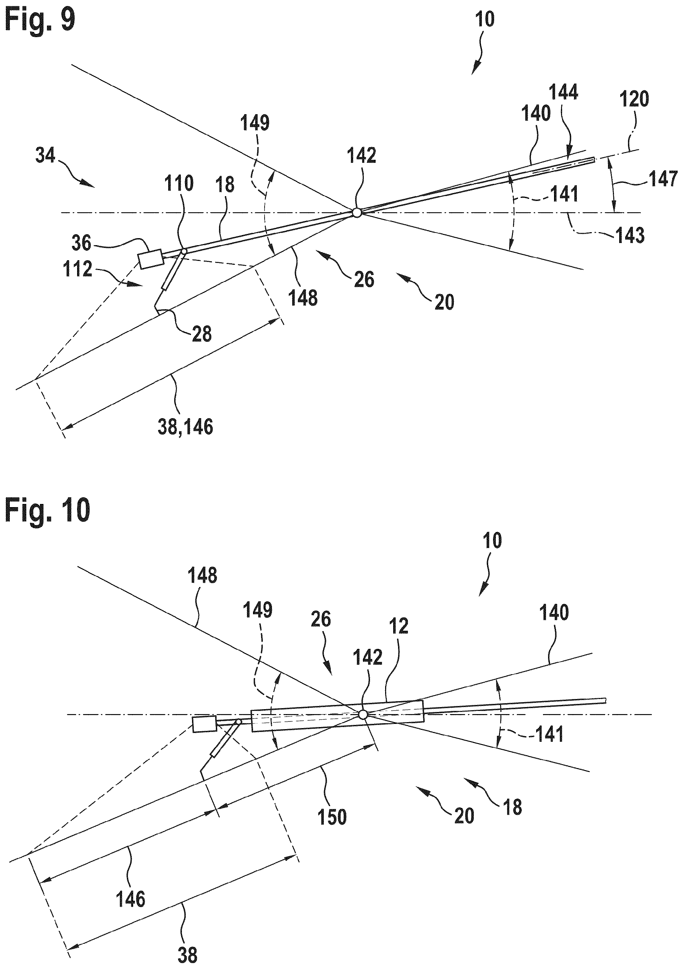

In der Fig. 9 ist die Resektoskopvorrichtung 10 in einer weiteren schematischen Darstellung gezeigt. Die Winkelstellung des Trägerarms 26 entspricht der Winkelstellung gemäß Fig. 7. Diese wurde durch Wahl der Winkelstellung eingestellt. Der Abwinkelabschnitt 112 wurde also gegenüber dem distalen Endstück 34 abgewinkelt. In dieser Winkelstellung ist die Werkzeugspitze 28 auf einer Oberfläche eines Anwendungskonus 148 bewegbar. Zur Bewegung auf der Oberfläche des Anwendungskonus 148 kann der Benutzer einen proximalen Abschnitt 144 des Schafts 18 innerhalb eines Manipulationskonus 140 bewegen. Dies durch ein Schwenken um einen Schwenkpunkt 142 und ein lineares Verschieben des Schafts 18 durch den Schwenkpunkt 142. Bewegt der Benutzer etwa die Werkzeugspitze 28 näher zum Schwenkpunkt 142, so schwenkt er den Schaft 18 derart, dass ein Winkel 147 zwischen der Längsachse 120 des Schafts 18 und einer Mittelachse 143 des Manipulationskonus 140 kleiner wird. In die entgegengesetzte Richtung vergrößert er entsprechend den Winkel 147. Eine maximale Entfernung der Werkzeugspitze 28 zum Schwenkpunkt 142 auf der Oberfläche des Anwendungskonus 148 wird entsprechend durch einen Öffnungswinkel 141 des Manipulationskonus 140 bei der Wahl der Winkelstellung des Trägerarms 26 begrenzt. Da das Resektionswerkzeug 20 gemeinsam mit dem Schaft 18 um die Längsachse 120 des Schafts 18 drehbar ist, weisen der Anwendungskonus 148 und der Manipulationskonus 140 die Form eines Kreiskegels auf. Entsprechend entspricht der Öffnungswinkel 141 einem Zweifachen des Winkels 147.

Zudem definiert die Bilderfassungseinrichtung 36 im distalen Endstück 34, wie oben beschrieben, den Beobachtungsbereich 38. Dieser schneidet bereichsweise den Anwendungskonus 148. In diesem Bereich, einem Oberflächenabschnitt 146 des Anwendungskonus 148, ist die Werkzeugspitze bewegbar. Genauer ist die Werkzeugspitze 28 durch Manipulation des proximalen Abschnitts 144 des Schafts 18 innerhalb des Manipulationskonus 140 und Wahl der Winkelstellung des Trägerarms 26 auf dem innerhalb des Beobachtungsbereichs 38 befindlichen Oberflächenabschnitt 146 des Anwendungskonus 148 bewegbar. Im dargestellten Fall wird der Oberflächenabschnitt 146 durch den Beobachtungsbereich 38 definiert. Das heißt, die Werkzeugspitze 28 könnte auch außerhalb des Beobachtungsbereichs 38 bewegbar sein.

Aufgrund der Winkelstellung des Trägerarms 26 ist der Öffnungswinkel 149 des Anwendungskonus 148 größer als der Öffnungswinkel 141 des Manipulationskonus 140. Da die Werkzeugspitze 28 über den Querschnitt des Schafts 18 hinaus abgewinkelt wurde, kann bei kleinerer Schwenkung des Schafts 18 weiter entferntes Gewebe erreicht werden im Vergleich zu einem nicht-abwinkelbaren Trägerarm (nicht dargestellt). Der Öffnungswinkel 149 des Anwendungskonus 148 ist wenigstens 1,5-mal so groß ist wie der Öffnungswinkel 141 des Manipulationskonus 140, im dargestellten Fall wenigstens 2-mal so groß.

In Fig. 10 ist die Resektoskopvorrichtung 10 in einer schematischen Darstellung gezeigt, wobei die Resektoskopvorrichtung 10 in einer anderen Stellung als gemäß Fig. 8 angeordnet ist. Zudem erkennt man die Einführhülse 12, durch die sich der Schaft 18 und das Resektionswerkzeug 20 erstrecken. Der Manipulationskonus 140, der Anwendungskonus 148 und die Öffnungswinkel 141, 149 entsprechen denen gemäß Fig. 9. Ebenso ist die Winkelstellung dieselbe. Man sieht, dass der Schwenkpunkt 142 innerhalb der Einführhülse 12 angeordnet ist. Aufgrund der Winkelstellung des Trägerarms 26 kann das Resektionswerkzeug 20 nicht weiter nach proximal zurückgezogen werden. Entsprechend ist der abfahrbare Oberflächenabschnitt 146 durch die Einführhülse 12 und die Wahl der Winkelstellung begrenzt. In der gezeigten Ausführungsform ist ein Abstand 150 des Oberflächenabschnitts 146 des Anwendungskonus 148 zum Schwenkpunkt 142 wenigstens 2-mal so groß ist wie die Länge 152 des Abwinkelabschnitts 112 (siehe Fig. 7). Der Beobachtungsbereich 38 ragt also über den Oberflächenabschnitt 146 nach proximal hinaus.

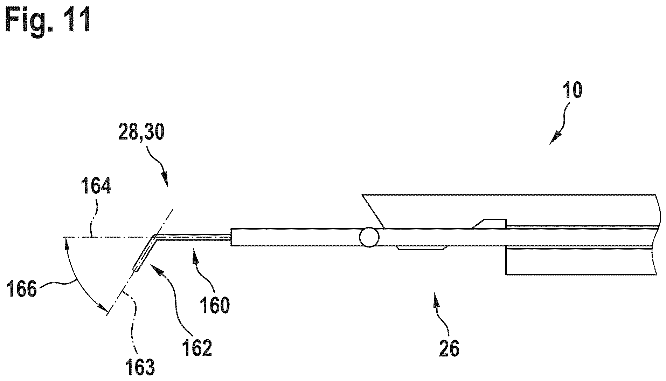

Fig. 11 zeigt eine schematische Darstellung eines distalen Abschnitts der Resektoskopvorrichtung 10 in einer Seitenansicht. Der Trägerarms 26 befindet sich in der Zuführungskonfiguration. Die Werkzeugspitze 28 ist als HS-Resektionsschlinge 30 ausgebildet, wie es beispielsweise in der Fig. 8 genauer zu sehen ist. Die HF-Resektionsschlinge 30 umfasst einen Zuleitungsabschnitt 160 und einen Arbeitsabschnitt 162. Der Arbeitsabschnitt 162 wird bei der Resektion für den Gewebeabtrag mit dem Gewebe in Kontakt gebracht. Der Zuleitungsabschnitt 160 verbindet den Arbeitsabschnitt 162 mit dem Trägerarm 26. Der Arbeitsabschnitt 162, insbesondere eine Längsachse 163 des Arbeitsabschnitts 162, ist relativ zu einer Längsachse 164 des Zuleitungsabschnitts 160 um einen Schlingenwinkel 166 abgewinkelt ist, der höchstens 90° beträgt. In der gezeigten Ausführungsform beträgt der Schlingenwinkel 166 45°. Der Arbeitsabschnitt 162 erstreckt sich also teilweise distal von dem Zuleitungsabschnitt 160. Dadurch kann in der gezeigten Zuführungskonfiguration eine Gewebestelle der Harnblase gegenüber des Blasenhalses leicht erreicht werden. Da der Trägerarm 26 abwinkelbar ist, können jedoch auch andere Gewebestellen leicht erreicht werden und die Werkzeugspitze 28 anwendungsspezifisch durch eine Wahl der Winkelstellung angeordnet werden.

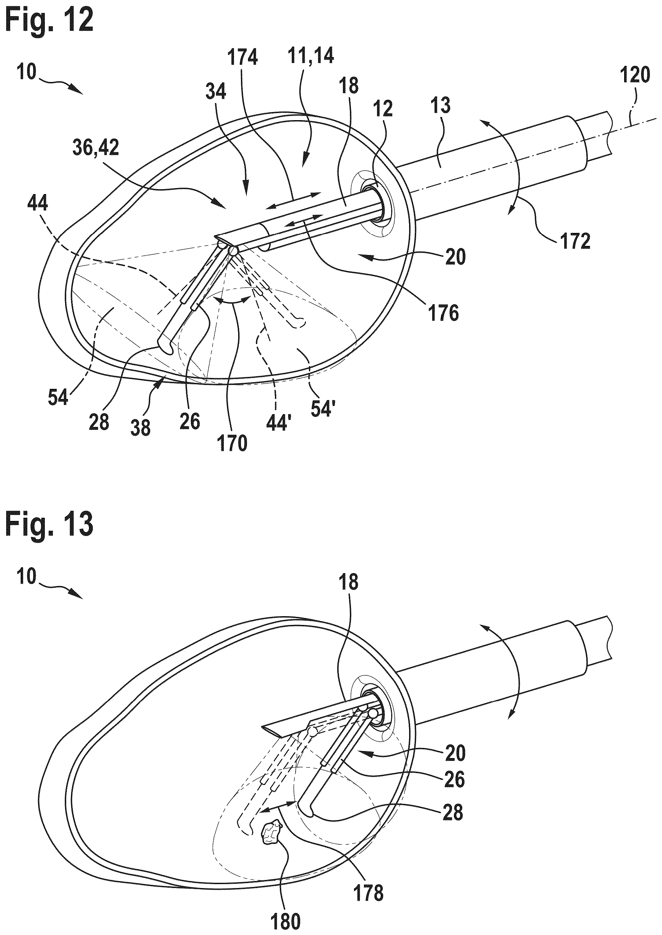

Fig. 12 zeigt eine weitere schematische Darstellung der Resektoskopvorrichtung 10. Diese ist in einem in die Kavität 14, im dargestellten Fall die Harnblase 11, teilweise eingeführten Zustand gezeigt. Der Schaft 18 und das Resektionswerkzeug 20 erstrecken sich durch die Einführhülse 12, welche in der Harnröhre 13 angeordnet ist, und das distale Endstück 34 mit der Bilderfassungseinrichtung 36 ragt in die Harnblase 11 hinein. Das Resektionswerkzeug 20 ist in zwei verschiedenen Winkelstellungen dargestellt. Beide definieren eine Anwendungskonfiguration. In den beiden verschiedenen Winkelstellungen ist die Werkzeugspitze 28 in unterschiedlichen Beobachtungsteilbereichen 54, 54' angeordnet. Die Beobachtungsteilbereiche 54, 54' sind jeweils unterschiedlichen Bilderfassungseinheiten 42 der Bilderfassungseinrichtung 36 zugeordnet, wobei die Bilderfassungseinheiten 42 unterschiedliche Blickrichtungen 44, 44' definieren (siehe auch Fig. 1). In der ersten Winkelstellung (durchgezogene Linien) ist die Werkzeugspitze 28 in einem teilweise nach distal gerichtetem Beobachtungsteilbereich 54 angeordnet. In der zweiten Winkelstellung (gestrichelte Linien) ist die Werkzeugspitze 28 in einem Beobachtungsteilbereich 54' angeordnet, das einer Bilderfassungseinheit 42 mit einer senkrecht zur Längsachse 120 des Schafts 18 angeordneten Blickrichtung 44' zugeordnet ist. Die Werkzeugspitze 28 ist durch Abwinkelung des Trägerarms 26 bzw. durch Wahl einer Winkelstellung von dem ersten Beobachtungsteilbereich 54 in den zweiten Beobachtungsteilbereich 54' bewegbar. Die Werkzeugspitze 28 ist also durch die Abwinkelung des Trägerarms 26 zwischen den Beobachtungsteilbereichen 54, 54' bewegbar.

Der Benutzer kann also die Werkzeugspitze 28 in Richtung des Pfeils 170 durch die Abwinkelung des Trägerarms 26 positionieren. Ferner kann der Benutzer die Werkzeugspitze 28 in Richtung des Pfeils 172 durch ein Schwenken des Schafts 18 und in Richtung des Pfeils 176 durch ein Vorschieben des Resektionswerkzeus 20 positionieren. Den Schaft 18 kann er in Richtung des Pfeils 174 positionieren, beispielsweise zur Ausrichtung der Bilderfassungseinrichtung 36. Ferner kann er den Schaft 18 gemeinsam mit dem Resektionswerkzeus 20 um die Längsachse 120 drehen (nicht dargestellt).

Anhand der Figuren 13 bis 18 wird im Folgenden erläutert, wie der Benutzer eine Resektion mittels des Resektionswerkzeus 20 mit abwinkelbaren Trägerarm 26 durchführen kann. Grundsätzlich sind zwei Gewebeabtragungsbewegungen denkbar (nicht abschließend). Einerseits könnte das Gewebe vorwiegend durch Längsverschiebung des Resektionswerkzeus 20, gemeinsam mit dem Schaft 18 oder unabhängig von diesem, abgetragen werden (Fig. 13). Bei dieser Gewebeabtragungsbewegung wird eine Winkelstellung des Trägerarms 26 einmalig vor Bewegungsdurchführung gewählt und anschließend beibehalten. Die Gewebeabtragungsbewegung entspricht somit annähernd jener, die bei einer herkömmlichen Resektion durchgeführt wird. Im Unterschied muss der Schaft 18 jedoch nicht so stark geschwenkt werden, da durch die Wahl der Winkelstellung abgelegene Gewebestellen bei geringer Schwenkung des Schafts 18 erreicht werden können.

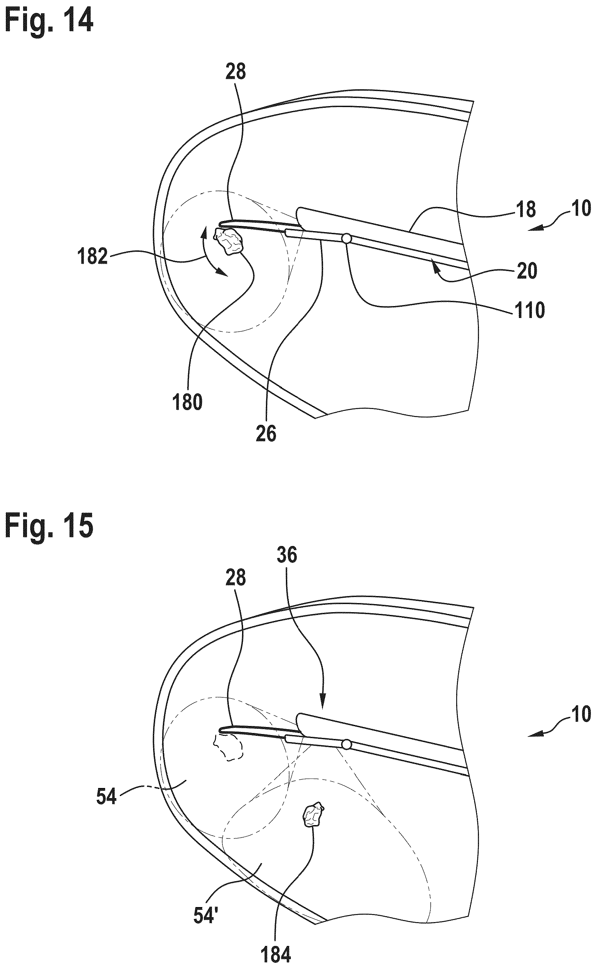

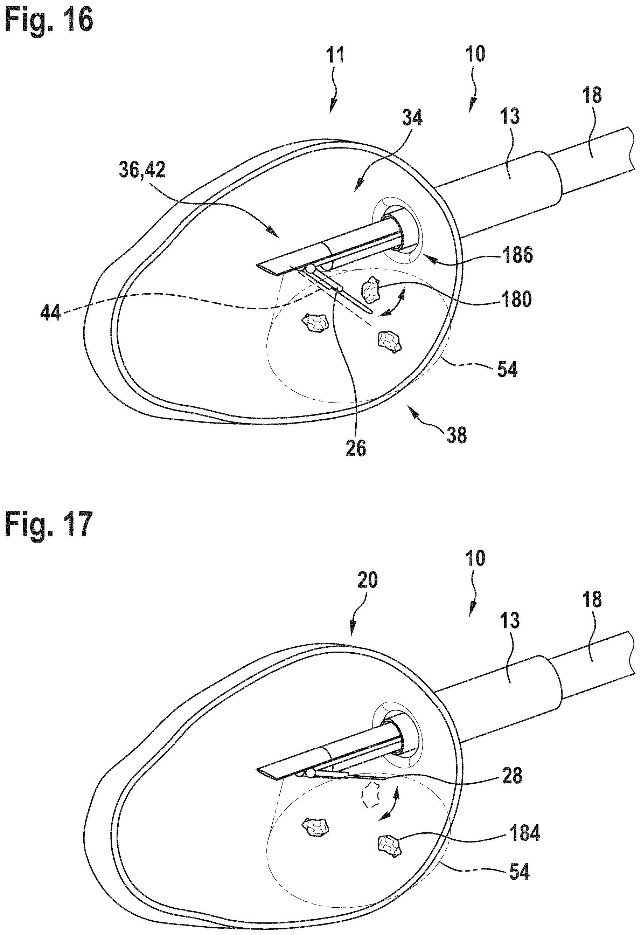

Andererseits könnte das Gewebe durch eine Schlingenbewegung des Abwinkelabschnitts 112 abgetragen werden (Fig. 14 und Fig. 16). Bei der Schlingenbewegung wird der Trägerarm 26 kontinuierlich abgewinkelt. Die Gewebeabtragungsbewegung ist in diesem Fall also eine Rotationsbewegung des Trägerarms 26 und keine Linearbewegung des Resektionswerkzeugs 20.

Ebenso denkbar ist eine Kombination beider Gewebeabtragungsbewegungen. Beispielsweise könnte eine erste Gewebestelle erst mittels der Linearbewegung und anschließend eine zweite Gewebestelle mittels der Schlingenbewegung bearbeitet werden.

Die Fig. 13 zeigt eine weitere schematische Darstellung der Resektoskopvorrichtung 10, wobei zwei verschiedene Linearstellungen des Resektionswerkzeugs 20 dargestellt sind. Diese Linearstellungen werden durch ein relatives Verschieben des Resektionswerkzeugs 20 zum Schaft 18 erreicht. Die Winkelstellung des Trägerarms 26 bleibt bei beiden Linearstellungen erhalten. Durch ein Bewegen zwischen den beiden Linearstellungen wird eine Bewegung der Werkzeugspitze 28 entlang der Richtung des Pfeils 178 erreicht. So kann die Werkzeugspitze 28 iterativ über zu entfernendes Gewebe 180 gefahren werden und diese Schicht für Schicht entfernt werden.

Die Fig. 14 zeigt eine weitere schematische Darstellung der Resektoskopvorrichtung 10. Durch ein kontinuierliches Verändern der Winkelstellung des Trägerarms 26 wird die Schlingenbewegung durchgeführt. Während dieser verbleiben das Resektionswerkzeug 20 proximal des Gelenks 110 und der Schaft 18 ortsfest zueinander. Durch das kontinuierliche Abwinkeln wird die Werkzeugspitze 28 also iterativ über das zu entfernende Gewebe 180 entlang der Richtung des Pfeils 182 bewegt. Ein schichtweiser Gewebeabtrag wird entsprechend durch die Schlingenbewegung ermöglicht.

Die Fig. 15 zeigt eine weitere schematische Darstellung der Resektoskopvorrichtung 10, wobei das Gewebe 180 gemäß Fig. 14 entfernt wurde. Da die Bilderfassungseinrichtung 36 die mehreren Beobachtungsteilbereiche 54 definiert, kann ein großer Teil des Harnblaseninnenraums überwacht werden, ohne dass ein erneutes Positionieren der Bilderfassungseinrichtung 36 notwendig ist. Wie dargestellt, kann sich etwa in dem Beobachtungsteilbereich 54' weiteres zu entfernendes Gewebe 184 befinden. Dieses kann ohne Bewegung der Bilderfassungseinrichtung 36 nach der Entfernung des Gewebes 180 (siehe Fig. 14) vom Benutzer identifiziert werden. Gemäß den Erläuterungen z.B. bezüglich Fig. 12 kann entsprechend die Werkzeugspitze 28 in den Beobachtungsteilbereich 54' bewegt werden. Hier kann anschießend entweder mittels der Linearbewegung oder der Schlingenbewegung das Gewebe 184 abgetragen werden.

Die Fig. 16 zeigt eine weitere schematische Darstellung der Resektoskopvorrichtung 10. Die Bilderfassungseinrichtung 36 umfasst eine Bilderfassungseinheit 42 mit einer Blickrichtung 44, die teilweise nach proximal gerichtet ist. Dadurch ist das distale Endstück 34 des Schafts 18 derart für die Durchführung der Resektion innerhalb einer Harnblase 11 anordenbar, dass sich der Blasenhals 186 innerhalb des Beobachtungsbereichs 38 der Bilderfassungseinrichtung 36 befindet. Entsprechend kann auch in einem Nahbereich um den Blasenhals 186 bzw. die Harnröhre 13 eine Resektion durchgeführt werden. Dies stellt eine besonders anspruchsvolle Resektion dar. Dennoch kann diese schonend für den Patienten durchgeführt werden, indem die Schlingenbewegung des Trägerarms 26 zum Gewebeabtrag verwendet wird. Ein starkes Schwenken des Schafts 18 ist nicht notwendig.

Nach erfolgtem Gewebeabtrag kann weiteres zu entfernendes Gewebe 184 im Beobachtungsbereich 54 identifiziert werden, wie es in der Fig. 17 dargestellt ist, welche eine weitere schematische Darstellung der Resektoskopvorrichtung 10 zeigt. Durch ein Drehen des Schafts 18 gemeinsam mit dem Resektionswerkzeug 20 kann die Werkzeugspitze 28 neu ausgerichtet werden, um das weitere zu entfernende Gewebe 184 zu erreichen.

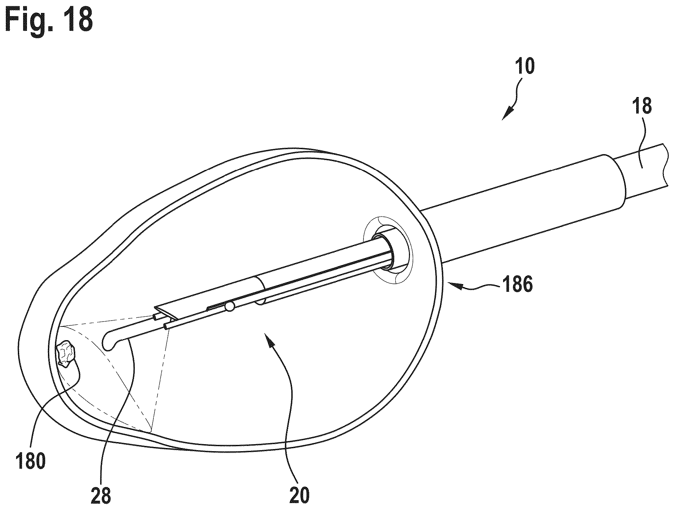

Die Fig. 18 zeigt eine weitere schematische Darstellung der Resektoskopvorrichtung 10. Das Resektionswerkzeug 20 ist in der Zuführungskonfiguration dargestellt. Aufgrund des Schlingenwinkels 166 von höchsten 90° (siehe Fig. 11 und zugehörige Beschreibung) kann in der Zuführungskonfiguration zu entfernendes Gewebe 180 erreicht werden, das dem Blasenhals 186 gegenüberliegt. Dazu muss der Schaft 18 aufgrund des Schlingenwinkels 166 nicht stark geschwenkt werden.

### Bezugszeichenliste

- 10: Resektoskopvorrichtung
- 11: Harnblase
- 12: Einführhülse
- 13: Harnröhre
- 14: Kavität
- 15: distales Endstück
- 16: Längsachse
- 17: Führungskanal
- 18: Schaft
- 19: Grundkörper
- 20: Resektionswerkzeug
- 22: Gewebe
- 24: Führungseinrichtung
- 26: Trägerarm
- 27: weiterer Trägerarm
- 28: Werkzeugspitze
- 30: HF-Resektionsschlinge
- 32: Spülvorrichtung
- 33: Zuleitung
- 34: distales Endstück
- 36: Bilderfassungseinrichtung
- 38: Beobachtungsbereich
- 40: Auslauf
- 42: Bilderfassungseinheit
- 43: Eingangsoptik
- 44: Blickrichtung
- 46: Beleuchtungseinrichtung
- 47: Beleuchtungseinheit
- 48: Rücklauf
- 49: Spalt
- 50: proximale Bedienbaugruppe
- 51: Betätigungsvorrichtung
- 52: Sichtwinkel
- 54: Beobachtungsteilbereich
- 56: Überlappungsbereich
- 58: distales Ende
- 59: distale Endfläche
- 60: Resektoskop
- 62: Steuereinheit
- 64: Anzeigeeinrichtung
- 110: Gelenk
- 111: Drehachse
- 112: Abwinkelabschnitt
- 113: abwinkelbarer Abschnitt
- 114: proximaler Abschnitt
- 116: Haupterstreckungsachse
- 118: Armwinkel
- 120: Längsachse
- 122: Betätigungsmechanismus
- 123: Linie
- 130: Abstand
- 132: Längsachse
- 134: Radius
- 136: Querschnittsprofil
- 140: Manipulationskonus
- 141: Öffnungswinkel
- 142: Schwenkpunkt
- 143: Mittelachse
- 144: proximaler Abschnitt
- 146: Oberflächenabschnitt
- 148: Anwendungskonus
- 149: Öffnungswinkel
- 150: Abstand
- 152: Länge
- 160: Zuleitungsabschnitt
- 162: Arbeitsabschnitt
- 163: Längsachse
- 164: Längsachse
- 166: Schlingenwinkel
- 170: Pfeil
- 172: Pfeil
- 174: Pfeil
- 176: Pfeil
- 178: Pfeil
- 180: zu entfernendes Gewebe
- 182: Pfeil
- 184: weiteres zu entfernendes Gewebe
- 186: Blasenhals

## Patentansprüche

1. Resektoskopvorrichtung (10), umfassend:
einen Schaft (18);
eine Bilderfassungseinrichtung (36), die an einem distalen Endstück (34) des Schafts (18) angeordnet ist und einen Beobachtungsbereich (38) definiert;
ein Resektionswerkzeug (20) mit einer Werkzeugspitze (28) und einem Trägerarm (26); und
ein Gelenk (110), insbesondere ein mechanisches Drehgelenk, mittels dessen der Trägerarm (26) gelenkig abwinkelbar ist;
wobei in zumindest einer Winkelstellung des Trägerarms (26) ein Abstand (130) zwischen der Werkzeugspitze (28) und einer Längsachse (132) des distalen Endstücks (34) einen Radius (134) eines Querschnittsprofils (136) des distalen Endstücks (34) um wenigstens einen Faktor 2 überschreitet.

2. Resektoskopvorrichtung (10), insbesondere nach Anspruch 1, umfassend:
einen Schaft (18);
eine Bilderfassungseinrichtung (36), die an einem distalen Endstück (34) des Schafts (18) angeordnet ist und einen Beobachtungsbereich (38) definiert;
ein Resektionswerkzeug (20) mit einer Werkzeugspitze (28) und einem Trägerarm (26); und
ein Gelenk (110), insbesondere ein mechanisches Drehgelenk, mittels dessen der Trägerarm (26) gelenkig abwinkelbar ist;
wobei der Schaft (18) einen Manipulationskonus (140) definiert, innerhalb dessen ein bezüglich eines Schwenkpunkts (142) proximaler Abschnitt (144) des Schafts (18) von einem Benutzer manipulierbar ist,
wobei die Werkzeugspitze (28) durch Manipulation des proximalen Abschnitts (144) des Schafts (18) innerhalb des Manipulationskonus (140) und Wahl zumindest einer Winkelstellung des Trägerarms (26) auf einem innerhalb des Beobachtungsbereichs (38) befindlichen Oberflächenabschnitt (146) eines Anwendungskonus (148) bewegbar ist,
wobei ein Öffnungswinkel (149) des Anwendungskonus (148) wenigstens 1,5-mal so groß ist wie ein Öffnungswinkel (141) des Manipulationskonus (140).

3. Resektoskopvorrichtung (10) nach Anspruch 2,
wobei das Resektionswerkzeug (20) einen Abwinkelabschnitt (112) definiert, der mittels des Gelenks (110) abwinkelbar ist, und
wobei ein Abstand (150) des Oberflächenabschnitts (146) des Anwendungskonus (148) zum Schwenkpunkt (142) wenigstens 2-mal so groß ist wie die Länge (152) des Abwinkelabschnitts (112).

4. Resektoskopvorrichtung (10), insbesondere nach einem der vorhergehenden Ansprüche,
umfassend:
einen Schaft (18);
eine Bilderfassungseinrichtung (36), die an einem distalen Endstück (34) des Schafts (18) angeordnet ist und einen Beobachtungsbereich (38) definiert;
ein Resektionswerkzeug (20) mit einer Werkzeugspitze (28) und einem Trägerarm (26); und
ein Gelenk (110), mittels dessen der Trägerarm (26) gelenkig abwinkelbar ist;
wobei die Bilderfassungseinrichtung (36) zumindest zwei Bilderfassungseinheiten (42) umfasst, die in unterschiedliche Blickrichtungen (44) gerichtet sind,
wobei die Bilderfassungseinheiten (42) jeweils einen Beobachtungsteilbereich (54) des Beobachtungsbereichs (38) definieren, und
wobei die Werkzeugspitze (28) durch die Abwinkelung des Trägerarms (26) zwischen den Beobachtungsteilbereichen (54) bewegbar ist.

5. Resektoskopvorrichtung (10) nach einem der vorhergehenden Ansprüche,
wobei der Trägerarm (26) mittels des Gelenks (110) relativ zum distalen Endstück (34) des Schafts (18) abwinkelbar ist, und
wobei das distale Endstück (34) relativ zu einem Grundkörper (19) des Schafts (18) unbeweglich ist.

6. Resektoskopvorrichtung (10) nach einem der vorhergehenden Ansprüche,
wobei der Schaft (18) und das Resektionswerkzeug (20) unabhängig voneinander entlang einer Längsachse (120) des Schafts (18) bewegbar sind.

7. Resektoskopvorrichtung (10) nach einem der vorhergehenden Ansprüche,
wobei das Resektionswerkzeug (20) einen Abwinkelabschnitt (112) definiert, der mittels des Gelenks (110) abwinkelbar ist, und
wobei eine Länge (152) des Abwinkelabschnitts (112) einen Radius (134) eines Querschnittsprofils (136) des distalen Endstücks (34) um wenigstens einen Faktor 2, insbesondere um wenigstens einen Faktor 3, überschreitet,
und / oder wobei die Länge (152) des Abwinkelabschnitts (112) zwischen 15 mm und 50 mm und insbesondere zwischen 25 mm und 35 mm

8. Resektoskopvorrichtung (10) nach einem der vorhergehenden Ansprüche, ferner umfassend:
eine Spülvorrichtung (32), mittels derer ein durch das Resektionswerkzeug (20) bearbeitbarer Bereich spülbar ist, umfassend eine Zuleitung (33), mittels derer eine Spülflüssigkeit entlang des Schafts (18) leitbar ist, wobei sich die Zuleitung (33) innerhalb des Trägerarms (26) erstreckt, und wobei insbesondere die Spülvorrichtung (32) einen Auslauf (40) umfasst, der an einem mittels des Gelenks (110) abwinkelbaren Abschnitt (113) des Trägerarms (26) angeordnet ist.

9. Resektoskopvorrichtung (10) nach einem der vorhergehenden Ansprüche,
wobei das Resektionswerkzeug (20) einen weiteren abwinkelbaren Trägerarm (26) umfasst,
wobei zwischen dem Trägerarm (26) und dem weiteren Trägerarm (27) die Werkzeugspitze (28) angeordnet ist, und
wobei sich insbesondere der Schaft (18) zumindest in einer Zuführungskonfiguration zwischen den abwinkelbaren Abschnitten (113) der Trägerarme (26, 27) erstreckt.

10. Resektoskopvorrichtung (10) nach einem der vorhergehenden Ansprüche,
wobei die Werkzeugspitze (28) eine HS-Resektionsschlinge (30) umfasst, die einen Zuleitungsabschnitt (160) und einen Arbeitsabschnitt (162) umfasst, wobei der Arbeitsabschnitt (162) relativ zu einer Längsachse (164) des Zuleitungsabschnitts (160) um einen Schlingenwinkel (166) abgewinkelt ist, der höchstens 90° beträgt, und wobei sich insbesondere der Arbeitsabschnitt (162) teilweise distal von dem Zuleitungsabschnitt (160) erstreckt.

11. Resektoskopvorrichtung (10) nach einem der vorhergehenden Ansprüche,
wobei das distale Endstück (34) des Schafts (18) derart für die Durchführung einer Resektion zumindest teilweise innerhalb einer Harnblase (11) anordenbar ist, dass sich der Blasenhals innerhalb des Beobachtungsbereichs (38) der Bilderfassungseinrichtung (36) befindet.

12. Resektoskopvorrichtung (10) nach einem der vorhergehenden Ansprüche,
wobei das Resektionswerkzeug (20) gemeinsam mit dem Schaft (18) um eine Längsachse (120) des Schafts (18) drehbar ist.

13. Resektoskopvorrichtung (10) nach einem der vorhergehenden Ansprüche, ferner umfassend:
eine Einführhülse (12), die einer Kavität (14) eines Patienten teilweise zuführbar ist und eine Längsachse (16) definiert,
wobei sich der Schaft (18) innerhalb der Einführhülse (12) erstreckt,
wobei der Schaft (18) und das Resektionswerkzeug (20) unabhängig voneinander relativ zu der Einführhülse (12) entlang der Längsachse (16) der Einführhülse (12) bewegbar sind, und
wobei insbesondere der Schaft (18) und das Resektionswerkzeug (20) gemeinsam mit der Einführhülse (12) der Kavität (14) zuführbar sind.

14. Resektoskopvorrichtung (10) nach einem der vorhergehenden Ansprüche,
ferner umfassend eine proximale Bedienbaugruppe (50), die durch einen Benutzer greifbar ist und mittels derer der Schaft (18) manipulierbar ist.

15. Resektionswerkzeug (20), insbesondere für eine Resektoskopvorrichtung (10) nach einem der vorhergehenden Ansprüche, umfassend:
eine Werkzeugspitze (28) und einen Trägerarm (26); und
ein Gelenk (110), mittels dessen der Trägerarm (26) gelenkig abwinkelbar ist,
wobei das Resektionswerkzeug (20) einen Abwinkelabschnitt (112) definiert, der mittels des Gelenks (110) abwinkelbar ist, und wobei die Länge (152) des Abwinkelabschnitts (112) zwischen 15 mm und 50 mm und insbesondere zwischen 25 mm und 35 mm beträgt.

16. Resektoskop (60) mit einer Resektoskopvorrichtung (10) nach einem der Ansprüche 1 bis 15.
